(19) Europäisches Patentamt
European Patent Office
Office européen des brevets

(11) Numéro de publication: **0 214 004 B1**

(12) # FASCICULE DE BREVET EUROPEEN

(45) Date de publication de fascicule du brevet: **31.03.93**

(21) Numéro de dépôt: **86401661.3**

(22) Date de dépôt: **24.07.86**

(51) Int. Cl.[5]: **C07D 417/12**, C07D 401/12, C07D 413/12, C07D 417/14, A61K 31/47, A61K 31/505, C07D 215/56, C07D 265/22, C07D 277/46, C07D 491/04, //(C07D491/04,307:00,221:00)

Le dossier contient des informations techniques présentées postérieurement au dépôt de la demande et ne figurant pas dans le présent fascicule.

(54) **Dérivés de l'acide 4-OH quinoléine carboxylique substitué en 2 par un groupement dihydroxylé éventuellement éthérifié ou estérifié, procédé et inter-médiaires de préparation, leur application comme médicaments et les compositions les renfermant.**

(30) Priorité: **25.07.85 FR 8511389**

(43) Date de publication de la demande: **11.03.87 Bulletin 87/11**

(45) Mention de la délivrance du brevet: **31.03.93 Bulletin 93/13**

(84) Etats contractants désignés: **AT BE CH DE FR GB IT LI LU NL SE**

(56) Documents cités:
**DE-A- 3 320 102**

**CHEMICAL ABSTRACTS, vol. 87, 1977, page 598, résumé no. 152226n, Columbus, Ohio, US**

**CHEMICAL ABSTRACTS, vol. 88, 1978, page 589, résumé no. 6900g, Columbus, Ohio, US**

(73) Titulaire: **ROUSSEL-UCLAF**
**35, Boulevard des Invalides**
**F-75007 Paris(FR)**

(72) Inventeur: **Clemence, François**
**2, rue Turgot**
**F-75009 Paris(FR)**
Inventeur: **Le Martret, Odile**
**42, avenue de Versailles**
**F-75016 Paris(FR)**
Inventeur: **Delevallee, Françoise**
**48-50, avenue de la Dame Blanche**
**F-94120 Fontenay-Sous-Bois(FR)**

(74) Mandataire: **Bourgouin, André et al**
**Département des Brevets ROUSSEL UCLAF**
**B.P. no 9**
**F-93230 Romainville (FR)**

EP 0 214 004 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

Le document DE-A-3320102 décrit des dérivés de l'acide 4-hydroxy 3-quinoléine carboxylique présentant une activité anti-inflammatoire.

La présente invention concerne de nouveaux dérivés de l'acide 4-OH 3-quinoléïne carboxylique substitués en 2 par un groupement comportant deux fonctions hydroxyle éventuellement éthérifiées ou estérifiées, leurs procédés de préparation, les nouveaux intermédiaires obtenus, leur application comme médicaments et les compositions les renfermant.

L'invention a pour objet les composés de formule (I) :

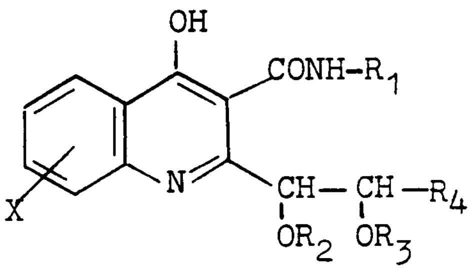

(I)

dans laquelle X en position 5, 6, 7 ou 8 représente un atome d'hydrogène, un atome d'halogène, un radical alcoyle, linéaire ou ramifié, renfermant de 1 à 5 atomes de carbone, un radical alcoxy, linéaire ou ramifié, renfermant de 1 à 4 atomes de carbone, un radical trifluorométhyle, un radical trifluorométhylthio ou un radical trifluorométhoxy, $R_1$ représente un radical choisi parmi les radicaux thiazolyle, 4,5-dihydrothiazolyle, pyridinyle, oxazolyle, isoxazolyle, imidazolyle, pyrimidyle et tétrazolyle, éventuellement substitués par un radical alcoyle renfermant de 1 à 4 atomes de carbone ou $R_1$ représente un radical phényle éventuellement substitué par un ou plusieurs radicaux choisis dans le groupe formé par le radical hydroxy, les radicaux alcoyles renfermant de 1 à 4 atomes de carbone, les radicaux alcoxy renfermant de 1 à 4 atomes de carbone, le radical trifluorométhyle, le radical nitro, et les atomes d'halogène, $R_2$ et $R_3$, identiques ou différents, représentent un atome d'hydrogène, un radical alcoyle renfermant de 1 à 4 atomes de carbone, un radical phényle, ou un radical

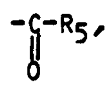

$R_5$ représentant un radical alcoyle renfermant de 1 à 4 atomes de carbone ou un radical phényle, ou $R_2$ et $R_3$ forment ensemble un reste d'acétonide, $R_4$ représente un atome d'hydrogène, un radical alcoyle renfermant de 1 à 4 atomes de carbone ou un radical phényle, ainsi que les sels d'addition des produits de formule (I) avec les acides et les bases.

Lorsque X représente un atome d'halogène, il s'agit de préférence d'un atome de chlore.

Lorsque X représente un radical alcoyle, il s'agit de préférence du radical méthyle, éthyle, n-propyle, n-butyle, n-pentyle, isopropyle ou isobutyle.

Lorsque X représente un radical alcoxy, il s'agit de préférence du radical méthoxy, éthoxy ou n-propoxy.

Lorsque $R_1$ représente un radical hétérocycle substitué par un radical alcoyle, il s'agit de préférence d'un radical hétérocyclique substitué par un radical méthyle ou éthyle.

Lorsque $R_1$ représente un radical phényle substitué, il s'agit de préférence d'un radical phényle substitué par au moins un radical choisi dans le groupe formé par le radical hydroxy, les radicaux méthyle et éthyle, les radicaux méthoxy et éthoxy, le radical trifluorométhyle, le radical nitro et l'atome de chlore.

Lorsque $R_2$ ou $R_3$ ou $R_4$ représentent un radical alcoyle, il s'agit de préférence du radical méthyle, éthyle, propyle, isopropyle, butyle ou isobutyle.

Lorsque $R_2$ ou $R_3$ représentent un radical

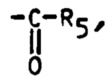

$R_5$ est de de préférence un radical méthyle, éthyle, n-propyle ou phényle.

Parmi les sels d'addition avec les acides, on peut citer ceux formés avec les acides minéraux tels que les acides chlorhydrique, bromhydrique, sulfurique ou phosphorique ainsi que ceux formés avec les acides sulfoniques tels que les acides alcoyles ou arylsulfoniques, par exemple, l'acide méthanesulfonique ou paratoluènesulfonique.

Parmi les sels d'addition avec les bases, on peut citer ceux formés avec les métaux alcalins comme le sodium et le potassium et les amines, par exemple, la triméthylamine ou la diméthylamine.

L'invention a notamment pour objet les composés pour lesquels X est en position 8 ainsi que leurs sels d'addition avec les acides et les bases et ceux pour lesquels X représente un radical trifluorométhyle, ainsi que leurs sels d'addition avec les acides et les bases.

L'invention a aussi notamment pour objet les composés pour lesquels X est en position 7 et représente un atome de chlore, ainsi que leurs sels d'addition avec les acides et les bases.

L'invention a plus particulièrement pour objet les composés pour lesquels $R_1$ représente le radical thiazolyle, ainsi que leurs sels d'addition avec les acides et les bases et ceux pour lesquels $R_2$ et $R_3$ représentent un atome d'hydrogène et $R_4$ représente un atome d'hydrogène, un radical alcoyle renfermant de 1 à 4 atomes de carbone ou un radical phényle, ainsi que leurs sels d'addition avec les acides et les bases.

On retient plus particulièrement les produits décrits dans les exemples et tout particulièrement :

- le 2-(1,2-dihydroxyéthyl) 4-hydroxy N-(2-thiazolyl) 8-trifluorométhyl 3-quinoléine carboxamide ainsi que ses sels d'addition avec les acides et les bases,
- le 2-(1,2-dihydroxy propyl) 4-hydroxy N-(2-thiazolyl) 8-trifluorométhyl 3-quinoléine carboxamide ainsi que ses sels d'addition avec les acides et les bases,
- le 2-[1,2-bis (1-oxopropoxy) éthyl] 4-hydroxy N-(2-thiazolyl) 8-trifluorométhyl 3-quinoléine carboxamide ainsi que ses sels d'addition avec les acides et les bases.

L'invention a également pour objet un procédé de préparation des composés de formule (I) caractérisé en ce que l'on fait réagir un composé de formule II :

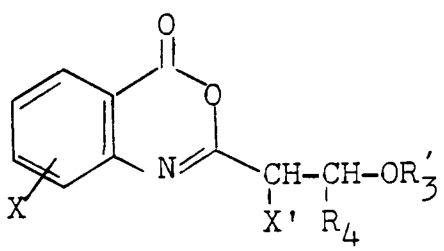

II

dans laquelle X et $R_4$ ont les significations déjà données, R'$_3$ est un radical alcoyle renfermant de 1 à 4 atomes de carbone ou un radical phényle, X' est un atome d'halogène,

- soit avec un composé de formule $III_A$ :

$CH_3$-CONH-$R_1$ $III_A$

dans laquelle $R_1$ a la signification précédente, pour obtenir un composé de formule IV :

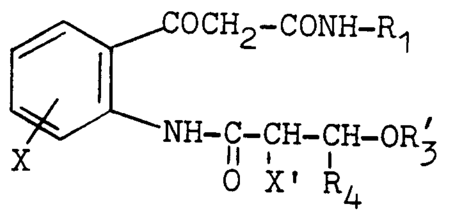

IV

- soit en présence d'une base avec un composé de formule $III_B$ :

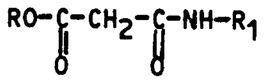

$III_B$

dans laquelle $R_1$ a la signification précédente et R est un radical alcoyle renfermant de 1 à 8 atomes de carbone, pour obtenir un composé de formule (A) :

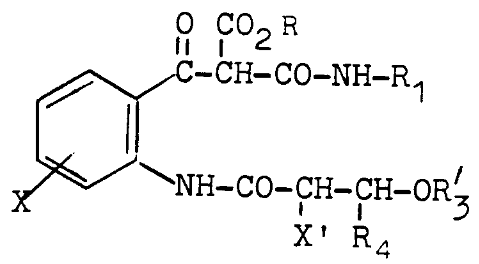

A

que l'on décarbalcoxyle pour obtenir un composé de formule IV précédemment défini, composé de formule IV ainsi obtenu que l'on cyclise en présence d'un agent alcalin pour obtenir un composé de formule V :

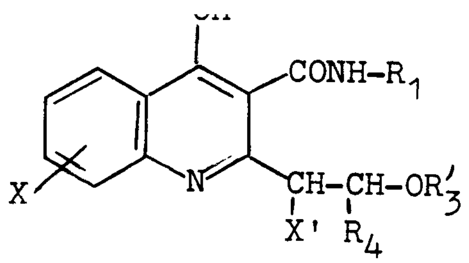

V

que l'on transforme en un composé de formule VI :

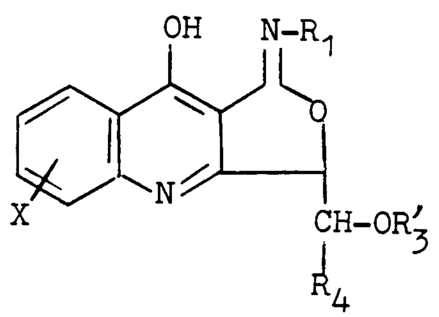

VI

composé de formule VI que l'on traite :

- soit par un agent d'hydrolyse acide pour obtenir un composé de formule (I) dans laquelle X, $R_1$ et $R_4$ ont les significations précédentes, $R_2$ est un atome d'hydrogène et $R_3$ un radical alcoyle renfermant de 1 à 4 atomes de carbone ou un radical phényle, que l'on transforme, si désiré, en composé de formule (I) dans laquelle X, $R_1$, $R_2$ et $R_4$ ont les significations données ci-dessus et $R_3$ représente un atome d'hydrogène, puis, le cas échéant, éthérifie ou estérifie une ou les deux fonctions hydroxyle, pour obtenir un composé de formule (I) dans laquelle $R_2$ et/ou $R_3$, identiques ou différents, représentent un radical alcoyle renfermant de 1 à 4 atomes de carbone, un radical phényle ou un groupement

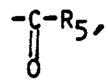

tel que défini précédemment, ou, le cas échéant, traite un composé de formule (I) dans laquelle $R_2$ et $R_3$ représentent un atome d'hydrogène par l'acétone en présence d'un agent acide, pour obtenir l'acétonide correspondant,
- soit par un acide de formule $R_5$-COOH, $R_5$ ayant la signification précédente, pour obtenir un produit de formule (I) dans laquelle X, $R_1$ et $R_4$ ont les significations précédentes, $R_2$ représente un radical

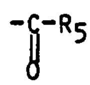

et $R_3$ un radical alcoyle renfermant de 1 à 4 atomes de carbone ou un radical phényle;
puis transforme les composés de formule (I) ainsi obtenus, si désiré, en sels par action d'un acide ou d'une base.

Dans les conditions préférentielles de mise en oeuvre de l'invention, le procédé ci-dessus décrit est réalisé de la manière suivante :

- X' est un atome de chlore dans le composé de formule II.
- la réaction entre le composé de formule II et le composé de formule $III_A$ a lieu en présence d'un organolithien ou d'un amidure de lithium, par exemple, en présence de butyllithium ou de diiosopropylamidure de lithium ; cette réaction s'effectue à basse température, de l'ordre de -70¤C.

Lorsque le composé de formule II réagit avec le composé de formule $III_B$, la base utilisée est, par exemple, l'hydroxyde de sodium et la réaction s'effectue à température ambiante.

Les conditions opératoires concernant cette étape du procédé et celles de la décarbalcoxylation du composé de formule A sont similaires à celles décrites dans le brevet européen N¤ 0141713.

La cyclisation du composé de formule IV est effectuée en présence d'un agent alcalin, tel qu'un hydrure ou un carbonate alcalin ou une amine, par exemple, en présence d'hydrure de sodium, de carbonate de sodium ou de potassium, de pipéridine, de 4-aminopyridine, de diméthyl aminopyridine, de triéthylamine, de 1,5-diazabicyclo[4.3.0]non-5-ène, de 1,4-diazabicyclo[2.2.2]octane ou de 1,5-diazabicyclo-[5.4.0]undec 5-ène.

Cette cyclisation est réalisée en présence d'un solvant, qui est de préférence le tétrahydrofuranne, mais d'autres solvants comme le diméthylformamide, le benzène, ou le toluène peuvent aussi être utilisés.

L'hydrolyse acide du composé de formule VI est effectuée de préférence par l'acide chlorhydrique, mais d'autres acides peuvent être utilisés, tel que l'acide sulfurique.

Le passage d'un composé de formule (I) dans laquelle $R_3$ est un radical alcoyle renfermant de 1 à 4 atomes de carbone ou phényle à un composé de formule (I) dans laquelle $R_3$ représente un atome d'hydrogène, peut par exemple être effectué en présence de l'iodure de triméthylsilane ou de tribromure de bore.

On peut opérer dans un solvant tel que l'acétonitrile ou le chlorure de méthylène.

Les réactions d'éthérification ou d'estérification des produits de formule (I) ayant une ou deux fonctions hydroxyle s'effectuent par des méthodes usuelles.

L'agent acide que l'on fait réagir lors de la formation de l'acétonide est par exemple l'acide paratoluènesulfonique.

L'invention concerne aussi un autre procédé de préparation des composés de formule (I) telle que définie précédemment, caractérisé en ce que l'on soumet un composé de formule $II_A$ :

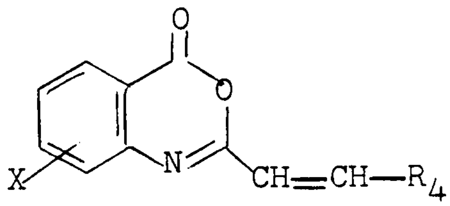

$II_A$

X et $R_4$ ayant les significations précédentes, à l'action d'un composé de formule $III_A$ :

$CH_3$-CONH-$R_1$ $III_A$

dans laquelle $R_1$ conserve sa signification précédente, pour obtenir un composé de formule $IV_A$ :

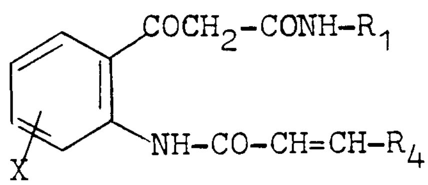

$IV_A$

que l'on cyclise en présence d'un agent alcalin pour obtenir un composé de formule $V_A$ :

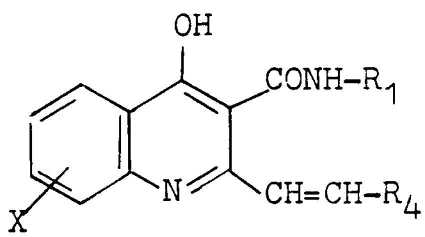

$V_A$

que l'on transforme en un composé de formule (I) dans laquelle X, $R_1$ et $R_4$ ont les significations données précédemment et $R_2$ et $R_3$ représentent un atome d'hydrogène composé de formule (I) dont on éthérifie ou estérifie éventuellement une ou les deux fonctions hydroxyle, pour obtenir un composé de formule (I) dans laquelle $R_2$ et/ou $R_3$ représentent soit un radical alcoyle renfermant de 1 à 4 atomes de carbone, un radical phényle, ou un radical

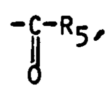

tel que défini précédemment, ou composés de formule (I) dans laquelle $R_2$ et $R_3$ représentent un atome d'hydrogène, que le cas échéant, l'on traite par l'acétone en présence d'un agent acide, pour obtenir l'acétonide correspondant, puis transforme, si désiré, les composés de formule (I) en sels par action d'un acide ou d'une base.

Le passage du composé de formule $II_A$ au composé de formule $IV_A$, puis la cyclisation de celui-ci en composé de formule $V_A$ s'effectuent dans les mêmes conditions préférentielles que celles décrites précédemment pour le premier procédé de l'invention.

La transformation du produit de formule $V_A$ en produit dihydroxylé de formule (I) s'effectue de préférence par action du permanganate de potassium, en présence du chlorure de triéthyl benzyl ammonium.

L'éthérification ou l'estérification d'une ou des deux fonctions hydroxyle du produit de formule (I) s'effectuent par des méthodes usuelles.

L'agent acide que l'on fait réagir lors de la formation de l'acétonide est par exemple, comme précédemment, l'acide paratoluène sulfonique.

Les composés de formule (I), tels que définis ci-dessous ainsi que leurs sels d'addition avec les acides et les bases présentent d'intéressantes propriétés pharmacologiques. Ce sont des composés doués de propriétés analgésique et anti-inflammatoire très actifs dans les modèles d'inflammation chronique.

Ces propriétés justifient leur application en thérapeutique, et l'invention a également pour objet, à titre de médicaments, les produits de formule (I), ainsi que leurs sels d'addition avec les acides et les bases pharmaceutiquement acceptables.

L'invention a plus particulièrement pour objet, à titre de médicaments :

- le 2-(1,2-dihydroxy éthyl) 4-hydroxy N-(2-thiazolyl) 8-trifluorométhyl 3-quinoléine carboxamide ainsi que ses sels d'addition avec les acides et les bases pharmaceutiquement acceptables, et
- le 2-(1,2-dihydroxy propyl) 4-hydroxy N-(2-thiazolyl) 8-trifluorométhyl 3-quinoléine carboxamide ainsi que ses sels d'addition avec les acides et les bases pharmaceutiquement acceptables.
- le 2-[1,2-bis (1-oxopropoxy) éthyl] 4-hydroxy N-(2-thiazolyl) 8-trifluorométhyl 3-quinoléine carboxamide ainsi que ses sels d'addition avec les acides et les bases.

Les médicaments, objets de la présente invention, peuvent être préconisés dans le traitement des maladies inflammatoires dégénératives telles que l'ostéoarthrose, des collagénoses diverses (tendinites, etc...), des maladies rhumatismales (la polyarthrite rhumatoïde, la spondylarthrite ankylosante), ainsi que dans le traitement d'autres maladies de nature auto-immune telles que le lupus érythémateux disséminé, les glomérulonéphrites, la sclérose en plaques.

Les médicaments, objets de l'invention, peuvent également être utilisés dans le traitement des algies musculaires, articulaires ou nerveuses, des douleurs dentaires, des migraines, du zona et également, à titre de traitement complémentaire dans les états infectieux et fébriles.

L'invention s'étend aux compositions pharmaceutiques renfermant comme principe actif, les médicaments définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous les formes pharmaceutiques couramment utilisées en médecine humaine comme, par exemple, les comprimés simples ou dragéifiés, les gélules, les granulés, les suppositoires, les préparations injectables, les pommades, les crèmes, les gels et les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie varie notamment en fonction de la voie d'administration, de l'affection traitée et du sujet en cause.

Par exemple, chez l'adulte, elle peut varier entre 20 mg et 2 g de principe actif par jour, par voie orale.

Les produits de formule II et $II_A$ peuvent être préparés par les procédés décrits dans le brevet européen 40573 ou dans Chemical Abstracts 1977, 87: 152226n et 1978, 88: 6900g.

Les produits de formule II, $II'_A$ correspondant à la formule $II_A$ dans laquelle $R_4$ a la signification indiquée précédemment à l'exception du radical phényle, les composés de formules IV, $IV_A$, V, $V_A$, A et VI sont des produits chimiques nouveaux ; l'invention a donc pour objet ces produits à titre de produits industriels nouveaux.

Les exemples suivants illustrent l'invention sans toutefois la limiter.

Exemple 1 : 4-hydroxy 2-(1-hydroxy 2-méthoxy éthyl) N-(2-thiazolyl) 8-(trifluorométhyl) 3-quinoléine carboxamide.

Stade A : 2-[(2-chloro 3-méthoxy 1-oxo propyl) amino]β-oxo N-(2-thiazolyl) 3-trifluorométhyl benzène propanamide.

On introduit à 0¤C, 350 cm3 de n-butyl lithium en solution dans l'hexane, dans une suspension de 34 g de 2-acétylamino thiazole dans 1100 cm3 de tétrahydrofuranne. On refroidit à -70/75¤C et ajoute une solution de 36,78 g de 2-(1-chloro 2-méthoxy éthyl) 8-trifluorométhyl 4 H-3,1-benzoxazine 4-one (produit préparé comme au stade A de l'exemple 12 du brevet européen 40 573 à partir d'acide 2-chloro 3-méthoxy propanoïque (Chem. Ber 92 1 081-7 (1959)) et d'acide 2-amino 3-trifluorométhyl benzoïque dans 250 cm3 de tétrahydrofuranne).

On verse la solution obtenue dans une solution aqueuse d'acide chlorydrique, extrait à l'éther, lave à l'acide chlorhydrique N, à l'eau, sèche, concentre sous pression réduite.

On empâte le résidu obtenu à l'éther, essore, lave à l'éther, sèche sous pression réduite et obtient 33,35 g de produit attendu fondant à 190¤C.

| Analyse : $C_{17}H_{15}N_3O_4F_3SCl$ : 449,845 | | | | | | |
|---|---|---|---|---|---|---|
| Calculé :<br>Trouvé : | C% 45,39<br>45,6 | H% 3,36<br>3,4 | N% 9,34<br>9,0 | F% 12,67<br>12,4 | Cl% 7,88<br>7,8 | S% 7,13<br>7,1 |

Stade B : 2-(1-chloro 2-méthoxy éthyl) 4-hydroxy N-(2-thiazolyl) 8-trifluorométhyl 3-quinoléine carboxamide.

On chauffe au reflux pendant 30 minutes 33,35 g de produit du stade A et 10 g de diméthylaminopyridine dans 300 cm3 de tétrahydrofuranne.

On refroidit à température ambiante, verse dans un mélange d'eau et d'acide chlorhydrique 2N, extrait à l'acétate d'éthyle, lave, séche, et concentre sous pression réduite. On empâte le résidu dans l'éther, essore, lave, sèche sous pression réduite à 60¤C et obtient 28,2 g de produit attendu fondant à 186¤C.

**Stade C** : 1,3-dihydro 3-(méthoxyméthyl) 1-[(2-thiazolyl) imino] 5-trifluorométhyl furo [3,4-b] quinoléin-9-ol.

On porte au reflux pendant 30 minutes 23,9 g de produit du stade B et 7,7 g de terbutylate de potassium dans 550 cm3 de dioxanne. On élimine le dioxanne sous pression réduite, reprend le résidu dans un mélange d'eau et d'acide chlorhydrique 2N, extrait l'insoluble avec un mélange d'acétate d'éthyle et de tétrahydrofuranne (80/20). On lave la phase organique à l'eau et réunit les phases aqueuses.

On neutralise la phase aqueuse par addition d'une solution aqueuse saturée de bicarbonate de sodium, extrait à l'acétate d'éthyle, lave à l'eau, sèche, et concentre sous pression réduite. On obtient 22 g de produit attendu.

**Stade D** : 4-hydroxy 2-(1-hydroxy 2-méthoxy éthyl) N-(2-thiazolyl) 8-(trifluorométhyl) 3-quinoléine carboxamide.

On agite pendant 16 heures à température ambiante une solution renfermant 22 g de produit du stade C, 70 cm3 d'eau, 130 cm3 d'acide chlorhydrique concentré.

On essore le précipité formé, le lave à l'eau, le reprend dans 200 cm3 d'eau, extrait avec un mélange d'acétate d'éthyle et de tétrahydrofuranne (50/50), lave la phase organique à l'eau, sèche, concentre sous pression réduite et obtient 15 g de produit que l'on purifie par chromatographie sur silice sous pression (éluant : acétate d'éthyle). On obtient 12,5 g de produit que l'on empâte dans l'éther, essore, lave à l'éther, et sèche sous pression réduite à 100¤C.

On obtient 11,83 g de produit attendu fondant à 216-218¤C.

Analyse : $C_{17}H_{14}O_4N_3F_3S$ : 413,384

| | | | | | |
|---|---|---|---|---|---|
| Calculé : | C% 49,39 | H% 3,41 | N% 10,16 | F% 13,76 | S% 7,76 |
| Trouvé : | 49,5 | 3,4 | 10,2 | 13,9 | 7,7 |

Exemple 2 : 2-(1,2-dihydroxy éthyl) 4-hydroxy N-(2-thiazolyl) 8-(trifluorométhyl) 3-quinoléine carboxamide.

On introduit lentement sous atmosphère inerte 6,7 cm3 d'iodure de triméthyl silane dans un mélange contenant 6,5 g de produit de l'exemple 1 et 120 cm3 d'acétonitrile, agite 16 heures à température ambiante, verse sur 400 cm3 d'eau additionnée de 50 cm3 de bisulfate de sodium.

On agite 45 minutes à température ambiante la suspension obtenue, ajoute 100 cm3 d'éther éthylique, agite 30 minutes, essore, lave à l'eau et sèche sous pression réduite à 75¤C pendant 16 heures. On obtient 5,9 g de produit que l'on dissout dans 75 cm3 de diméthylformamide, filtre, ajoute de l'éther au filtrat, glace, essore les cristaux obtenus, lave à l'éther, sèche sous pression réduite à 120¤C et obtient 4,86 g de produit attendu fondant à 255¤C.

**Analyse** : $C_{16}H_{12}N_3F_3O_4S$ : 399,356

| | C% | H% | N% | F% | S% |
|---|---|---|---|---|---|
| Calculé : | 48,12 | 3,03 | 10,52 | 14,27 | 8,03 |
| Trouvé : | 48,0 | 3,0 | 10,4 | 14,1 | 8,0 |

Exemple 3 : 2-(1,2-dihydroxypropyl) 4-hydroxy N-(2-thiazolyl) 8-trifluorométhyl 3-quinoléine carboxamide.
Stade A : β - oxo 2-[(1-oxo 2-butényl) amino] N-(2-thiazolyl) 3-trifluorométhyl benzène propanamide.

On opère comme indiqué au stade A de l'exemple 1 à partir de 19,44 g de 2-acétylamino thiazole et de 17,3 g de 2-(1-propényl) 8-trifluorométhyl 4 H-3,1-benzoxazin 4-one ; le produit est préparé comme au stade A de l'exemple 12 du brevet européen 40 573 à partir d'acide 2-amino 3-trifluorométhyl benzoïque et de chlorure de crotonyle.

On obtient 19,03 g de produit attendu fondant à 206-208¤C.

Stade B : 4-hydroxy 2-(1-propényl) N-(2-thiazolyl) 8-trifluorométhyl 3-quinoléine carboxamide.

On ajoute 17,5 g de produit du stade A en solution dans 175 cm3 de diméthyl acétamide, dans un mélange renfermant 2,11 g d'hydrure de sodium à 50 % en dispersion dans l'huile et 100 cm3 de diméthyl acétamide.

On chauffe à 120¤C et maintient 30 minutes à cette température.

On refroidit la solution, puis la verse sur un mélange d'eau et d'acide chlorhydrique 2N. On essore le précipité obtenu, le lave à l'eau, le sèche sous pression réduite à 80¤C et obtient 16,7 g de produit attendu fondant à 265¤C.

Stade C : 2-(1,2-dihydroxy propyl) 4-hydroxy N-(2-thiazolyl) 8-trifluorométhyl 3-quinoléine carboxamide.

On agite 1 heure à 0¤C, sous atmosphère inerte, le mélange renfermant 12,2 g de produit du stade B dans 300 cm3 de chlorure de méthylène, 9,16 g de chlorure de méthylbenzyl ammonium et 6,32 g de permanganate de potassium.

On ajoute 150 cm3 d'eau glacée, puis 150 cm3 d'une solution de bisulfite de sodium, essore l'insoluble obtenu, le lave à l'eau, le dissout partiellement dans le tétrahydrofuranne.

On sèche la solution organique, concentre sous pression réduite et obtient 5,8 g de produit brut, que l'on dissout dans du diméthylformamide tiède. On filtre la solution obtenue, concentre jusqu'à 40 cm3 environ, ajoute 60 cm3 d'éther éthylique, glace, essore les cristaux, les lave à l'éther, les sèche sous pression réduite à 100¤C. Le produit obtenu est dissous dans du tétrahydrofuranne.

On filtre, concentre sous pression réduite, empâte le résidu dans l'acétate d'éthyle, essore, lave, sèche sous pression réduite à 100¤C et obtient 3,04 g de produit attendu fondant à 275¤C.

**Analyse** : $C_{17}H_{14}N_3O_4F_3S$ : 413,384

| | | | | | |
|---|---|---|---|---|---|
| Calculé : | C% 49,39 | H% 3,41 | N% 10,16 | F% 13,79 | S% 7,76 |
| Trouvé : | 49,3 | 3,3 | 10,1 | 14,1 | 7,8 |

Exemple 4 : 2-[1,2-bis (1-oxopropoxy) éthyl] 4-hydroxy N-(2-thiazolyl) 8-trifluorométhyl 3-quinoléine carboxamide.

On mélange à 20¤C, 2,15 g du produit obtenu à l'exemple 2, 70 cm3 de chlorure de méthylène et 0,96 cm3 d'acide propionique. On ajoute après 5 minutes 2,9 g de dicyclohexylcarbodiimide puis 2,4 g de diméthylamino pyridine et maintient sous agitation pendant 1 heure 30 minutes. On filtre, lave le filtrat avec une solution aqueuse saturée de carbonate de sodium, avec une solution aqueuse d'acide chlorhydrique, puis à l'eau. On sèche, évapore à sec et recristallise le résidu dans l'acétonitrile. On obtient 1,34 g de produit attendu. F = 206¤C.

| | | | | | | |
|---|---|---|---|---|---|---|
| <u>Analyse</u> | : | $C_{22}H_{20}F_3N_3O_6S$ : 511,479 | | | | |
| Calculé | : | C% 51,66 | H% 3,94 | N% 8,22 | S% 6,27 | F% 11,14 |
| Trouvé | : | 51,7 | 3,9 | 8,1 | 6,2 | 11,1 |

<u>Exemple 5</u> : <u>2-[2-méthoxy 1-(1-oxopropoxy) éthyl] 4-hydroxy N-(2-thiazolyl) 8-trifluorométhyl 3-quinoléine carboxamide</u>.

On opère de manière analogue à celle décrite à l'exemple 4, en utilisant au départ 2 g de produit obtenu à l'exemple 1, 0,4 cm3 d'acide propionique, 1,2 g de dicyclohexylcarbodiimide et 0,3 g de diméthylaminopyridine. On obtient après recristallisation dans l'acétate d'éthyle 1 g de produit attendu. F = 190¤C.

| | | | | | | |
|---|---|---|---|---|---|---|
| <u>Analyse</u> | : | $C_{20}H_{18}F_3N_3O_5S$ : 469,442 | | | | |
| Calculé | : | C% 51,17 | H% 3,87 | F% 12,14 | N% 8,95 | S% 6,83 |
| Trouvé | : | 51,5 | 4,0 | 11,9 | 8,9 | 6,5 |

<u>Exemple 6</u> : <u>2-[1,2-(dibenzoyloxy) éthyl] 4-hydroxy N-(2-thiazolyl) 8-trifluorométhyl 3-quinoléine carboxamide</u>.

On opère de manière analogue à celle décrite à l'exemple 4, en utilisant au départ 0,5 g de produit obtenu a l'exemple 2, 0,37 g d'acide benzoïque, 0,67 g de dicyclohexylcarbodiimide et 0,075 g de diméthylaminopyridine. On obtient 0,8 g de produit brut que l'on dissout dans 15 cm3 de tétrahydrofuranne. On y ajoute 0,3 cm3 d'une solution 5,7 N d'acide chlorhydrique dans l'éthanol. On essore les cristaux formés et les dissout dans un mélange acétate d'éthyle-eau. On extrait à l'acétate d'éthyle, sèche et concentre à sec. On dissout le résidu dans du tétrahydrofuranne, ajoute de l'éther éthylique, refroidit à 0¤C pendant 2 heures, essore les cristaux et les sèche. On obtient 0,33 g de produit attendu. F = 240¤C.

| | | | | | | |
|---|---|---|---|---|---|---|
| <u>Analyse</u> | : | $C_{30}H_{20}F_3N_3O_6S$ : 607,568 | | | | |
| Calculé | : | C% 59,31 | H% 3,32 | N% 6,91 | F% 9,38 | S% 5,28 |
| Trouvé | : | 59,2 | 3,2 | 6,9 | 9,6 | 5,4 |

<u>Exemple 7</u> : <u>2-[1,2-(diacétyloxy) éthyl] 4-hydroxy N-(2-thiazolyl) 8-trifluorométhyl 3-quinoléine carboxamide</u>.

On opère de manière analogue à celle décrite à l'exemple 4, en utilisant au départ 2 g de produit obtenu à l'exemple 2, 0,7 cm3 d'acide acétique, 2,7 g de dicyclohexylcarbodiimide et 0,3 g de diméthylaminopyridine. On obtient 0,9 g de produit brut renfermant un peu de dicyclohexylurée que l'on élimine par lavage au tétrahydrofuranne, par recristallisation dans le diméthylformamide puis par formation du chlorhydrate dans le tétrahydrofuranne auquel on ajoute une solution éthanolique d'acide chlorhydrique. Après recristallisation dans l'acide acétique, on obtient 0,38 g de produit attendu. F = 270¤C.

| | | | | | | |
|---|---|---|---|---|---|---|
| <u>Analyse</u> | : | $C_{20}H_{16}F_3N_3O_6S$ : 483,432 | | | | |
| Calculé | : | C% 49,69 | H% 3,34 | N% 8,69 | F% 11,79 | S% 6,63 |
| Trouvé | : | 49,4 | 3,2 | 8,4 | 11,9 | 6,8 |

Exemple 8 : 2-(2,2-diméthyl 1,3 dioxolan-4-yl) 4-hydroxy N-(2-thiazolyl) 8-trifluorométhyl 3-quinoléine carboxamide.

On met en suspension 3 g de produit obtenu à l'exemple 2 dans 80 cm3 d'acétone. On porte au reflux puis ajoute 0,3 g d'acide paratoluène sulfonique et maintient au reflux pendant 5 heures. On refroidit à 20¤C, essore et sèche les cristaux sous pression réduite. On dissout dans 100 cm3 de tétrahydrofuranne en tiédissant à 40¤C, filtre, concentre à mi-volume, refroidit à 20¤C et ajoute de l'éther éthylique. On essore les cristaux, les lave à l'éther éthylique et les sèche. On obtient 1,2 g de produit attendu. F = 250¤C.

Analyse : $C_{19}H_{16}F_3N_3O_4S$ : 439,417

| | | | | | |
|---|---|---|---|---|---|
| Calculé : | C% 51,94 | H% 3,67 | N% 9,56 | F% 12,97 | S% 7,30 |
| Trouvé : | 51,8 | 3,5 | 9,4 | 12,8 | 7,1 |

Exemple 9 : 4-hydroxy 2-(1-hydroxy 2-méthoxyéthyl) N-(2-thiazolyl) 7-chloro 3-quinoléine carboxamide.

Stade A : 2-[(2-chloro 3-méthoxy 1-oxopropyl) amino] β-oxo N-(2-thiazolyl) 4-chloro benzène propanamide.

On opère de manière analogue à celle décrite au stade A de l'exemple 1, en utilisant au départ la 2-(1-chloro 2-méthoxyéthyl) 7-chloro 4H-3,1-benzoxazine 4-one (produit F = 82¤C préparé comme décrit au stade A de l'exemple 12 du brevet européen 40573 à partir d'acide 2-chloro 3-méthoxy propanoïque et d'acide 2-amino 4-chloro benzoïque). On obtient le produit attendu fondant à 180¤C avec un rendement de 76%.

Stade B : 2-(1-chloro 2-méthoxyéthyl) 4-hydroxy N-(2-thiazolyl) 7-chloro 3-quinoléine carboxamide.

On opère de manière analogue à celle décrite au stade B de l'exemple 1. Le produit est utilisé pour le stade suivant sans être isolé.

Stade C : 1,3-dihydro 3-(méthoxyméthyl) 1-[(2-thiazolyl) imino] 6-chloro furo [3,4-b] quinoléin-9-ol.

On opère de manière analogue à celle décrite au stade C de l'exemple 1, sans avoir isolé intermédiairement la quinoléine du stade B ci-dessus. On opère au reflux du tétrahydrofuranne pendant 24 heures. On obtient le produit attendu avec un rendement de 65%. F >270¤C.

Stade D : 4-hydroxy 2-(1-hydroxy 2-méthoxyéthyl) N-(2-thiazolyl) 7-chloro 3-quinoléine carboxamide.

On opère de manière analogue à celle décrite au stade D de l'exemple 1, en maintenant sous agitation dans l'acide chlorhydrique 6 N pendant 36 heures. On obtient le produit attendu. F > 270¤C.

Exemple 10 : 2-(1,2-dihydroxyéthyl) 4-hydroxy N-(2-thiazolyl) 7-chloro 3-quinoléine carboxamide.

Exemple de composition pharmaceutique :

Exemple 11 :

On a préparé des comprimés répondant à la formule suivante :

On a préparé des comprimés répondant à la formule suivante :

Produit de l'exemple 2 .................................... 50 mg

Excipient q.s. pour un comprimé terminé à ................ 350 mg

(détail de l'excipient : lactose, talc, amidon, stéarate de magnésium).

Etude pharmacologique

Activité anti-inflammamtoire : arthrite chronique à l'adjuvant (traitement préventif).

L'injection d'adjuvant de type Freund dans une patte postérieure provoque, chez le rat, l'apparition rapide d'une lésion inflammatoire primaire dans cette patte, puis, après un temps de latence de 13 à 15 jours, le déclenchement d'une arthrite secondaire affectant notamment l'autre patte postérieure. Le test est pratiqué sur des râts mâles âgés de 42 à 50 jours, qui reçoivent en injection intraplantaire, 0,1 ml d'adjuvant de type "Freund" (suspension dans l'huile de vaseline de 6 mg par ml de mycobacterium butyricum tués).

Les animaux reçoivent le produit étudié, par voie orale, du jour 0 (jour de l'injection de l'adjuvant) jusqu'à la veille du sacrifice, pratiqué le jour 17. Des animaux témoins arthritiques, des animaux témoins normaux ne reçoivent que le véhicule. Les critères d'appréciation de l'activité des substances étudiées sont les augmentations de volume des pattes postérieures injectées (inflammation primaire et secondaire) et non injectées (inflammation secondaire) par rapport au volume moyen des pattes correspondantes de témoins.

On détermine la $DA_{50}$, c'est-à-dire la dose qui diminue de 50 % les augmentations de volume des pattes postérieures des animaux traités par rapport aux animaux témoins.

Les $DA_{50}$ trouvées sont environ à 2 mg/Kg, 0,7 mg/Kg, 3 mg/Kg, 1 mg/kg et 5 mg/kg respectivement pour les produits des exemples 1, 2, 3, 4 et 5.

**Revendications**

**Revendications pour les Etats contractants suivants : BE, CH, DE, FR, GB, IT, LI, LU, NL, SE**

**1.** Les composés de formule (I) :

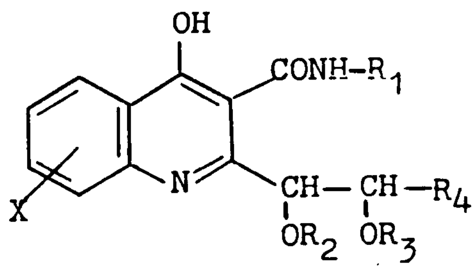

(I)

dans laquelle X en position 5, 6, 7 ou 8 représente un atome d'hydrogène, un atome d'halogène, un radical alcoyle, linéaire ou ramifié, renfermant de 1 à 5 atomes de carbone, un radical alcoxy, linéaire ou ramifié, renfermant de 1 à 4 atomes de carbone, un radical trifluorométhyle, un radical trifluorométhylthio ou un radical trifluorométhoxy, $R_1$ représente un radical choisi parmi les radicaux thiazolyle, 4,5-dihydrothiazolyle, pyridinyle, oxazolyle, isoxazolyle, imidazolyle, pyrimidyle et tétrazolyle, éventuellement substitués par un radical alcoyle renfermant de 1 à 4 atomes de carbone ou $R_1$ représente un radical phényle éventuellement substitué par un ou plusieurs radicaux choisis dans le groupe formé par le radical hydroxy, les radicaux alcoyles renfermant de 1 à 4 atomes de carbone, les radicaux alcoxy renfermant de 1 à 4 atomes de carbone, le radical trifluorométhyle, le radical nitro et les atomes d'halogène, $R_2$ et $R_3$, identiques ou différents, représentent un atome d'hydrogène, un radical alcoyle renfermant de 1 à 4 atomes de carbone, un radical phényle, ou un radical

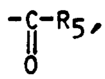

$R_5$ représentant un radical alcoyle renfermant de 1 à 4 atomes de carbone ou un radical phényle, ou $R_2$ et $R_3$ forment ensemble un reste d'acétonide, $R_4$ représente un atome d'hydrogène, un radical alcoyle renfermant de 1 à 4 atomes de carbone ou un radical phényle, ainsi que les sels d'addition des produits de formule (I) avec les acides et les bases.

**2.** Les composés de formule (I), tels que définis à la revendication 1, pour lesquels X est en position 8 ainsi que leurs sels d'addition avec les acides et les bases.

**3.** Les composés de formule (I), tels que définis à la revendication 1 ou 2, pour lesquels X représente un radical trifluorométhyle, ainsi que leurs sels d'addition avec les acides et les bases.

**4.** Les composés de formule (I), tels que définis à la revendication 1, pour lesquels X est en position 7 ainsi que leurs sels d'addition avec les acides et les bases.

**5.** Les composés de formule (I), tels que définis à la revendication 1 ou 4, pour lesquels X représente un atome de chlore, ainsi que leurs sels d'addition avec les acides et les bases.

**6.** Les composés de formule (I), tels que définis à l'une quelconque des revendications 1 à 5, pour lesquels $R_1$ représente le radical thiazolyle, ainsi que leurs sels d'addition avec les acides et les bases.

**7.** Les composés de formule (I) tels que définis à l'une quelconque des revendications 1 à 6, pour lesquels $R_2$ et $R_3$ représentent un atome d'hydrogène et $R_4$ représente un atome d'hydrogène, un radical alcoyle renfermant de 1 à 4 atomes de carbone ou un radical phényle, ainsi que leurs sels d'addition avec les acides et les bases.

**8.** L'un quelconque des composés de formule (I) dont les noms suivent :
- le 2-(1,2-dihydroxy éthyl) 4-hydroxy N-(2-thiazolyl) 8-trifluorométhyl 3-quinoléine carboxamide ainsi que ses sels d'addition avec les acides et les bases,
- le 2-(1,2-dihydroxy propyl) 4-hydroxy N-(2-thiazolyl) 8-trifluorométhyl 3-quinoléine carboxamide ainsi que ses sels d'addition avec les acides et les bases,
- le 2-[1,2-bis (1-oxopropoxy) éthyl] 4-hydroxy N-(2-thiazolyl) 8-trifluorométhyl 3-quinoléine carboxamide ainsi que ses sels d'addition avec les acides et les bases.

**9.** Procédé de préparation des composés de formule (I) telle que définie à la revendication 1 caractérisé en ce que l'on fait réagir un composé de formule II :

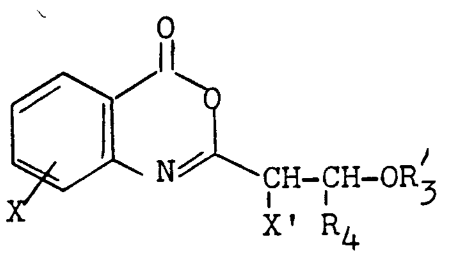

II

dans laquelle X et $R_4$ ont les significations déjà données, $R'_3$ est un radical alcoyle renfermant de 1 à 4 atomes de carbone ou un radical phényle, X' est un atome d'halogène,
- soit avec un composé de formule $III_A$ :

$CH_3$-$CONHR_1$ $III_A$

dans laquelle $R_1$ a la signification précédente, pour obtenir un composé de formule IV :

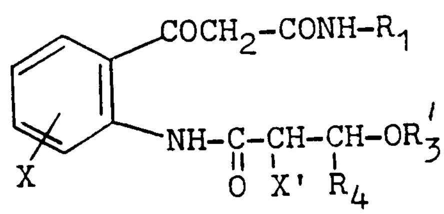

IV

- soit en présence d'une base avec un composé de formule $III_B$ :

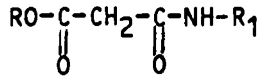

$III_B$

dans laquelle $R_1$ a la signification précédente et R est un radical alcoyle renfermant de 1 à 8 atomes de carbone, pour obtenir un composé de formule (A) :

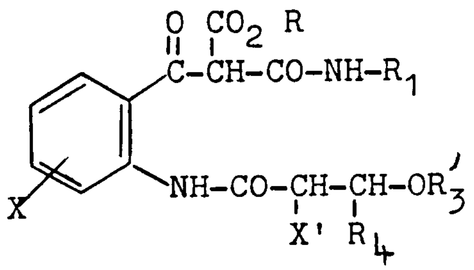

A

que l'on décarbalcoxyle pour obtenir un composé de formule IV précédemment défini, composé de formule IV ainsi obtenu que l'on cyclise en présence d'un agent alcalin pour obtenir un composé de formule V :

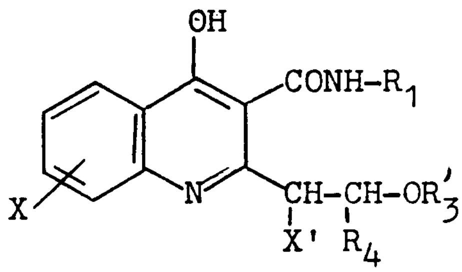

V

que l'on transforme en un composé de formule VI :

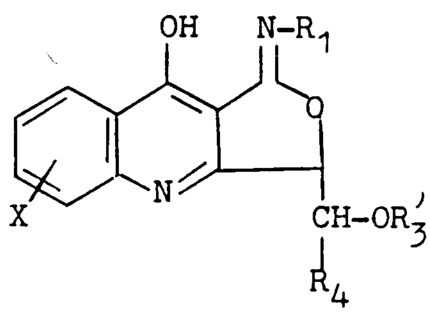

VI

composé de formule VI que l'on traite :

- soit par un agent d'hydrolyse acide pour obtenir un composé de formule (I) dans laquelle X, $R_1$ et $R_4$ ont les significations précédentes, $R_2$ est un atome d'hydrogène et $R_3$ un radical alcoyle renfermant de 1 à 4 atomes de carbone ou un radical phényle, que l'on transforme, si désiré, en composé de formule (I) dans laquelle X, $R_1$, $R_2$ et $R_4$ ont les significations données ci-dessus et $R_3$ représente un atome d'hydrogène, puis, le cas échéant, éthérifie ou estérifie une ou les deux fonctions hydroxyle, pour obtenir un composé de formule (I) dans laquelle $R_2$ et/ou $R_3$, identiques ou différents, représentent un radical alcoyle renfermant de 1 à 4 atomes de carbone, un radical phényle ou un groupement

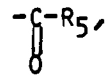

tel que défini précédemment, ou, le cas échéant, traite un composé de formule (I) dans laquelle $R_2$ et $R_3$ représentent un atome d'hydrogène par l'acétone en présence d'un agent acide, pour obtenir l'acétonide correspondant,

- soit par un acide de formule $R_5$-COOH, $R_5$ ayant la signification précédente, pour obtenir un produit de formule (I) dans laquelle X, $R_1$ et $R_4$ ont les significations précédentes, $R_2$ représente un radical

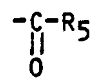

et $R_3$ un radical alcoyle renfermant de 1 à 4 atomes de carbone ou un radical phényle, composés de formule (I) ainsi obtenus, que l'on transforme, si désiré, en sels par action d'un acide ou d'une base.

**10.** Procédé de préparation des composés de formule (I) telle que définie à la revendication 1, caractérisé en ce que l'on soumet un composé de formule $II_A$ :

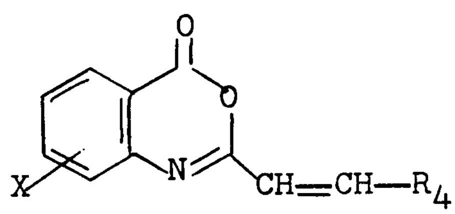

$II_A$

X et $R_4$ ayant les significations indiquées à la revendication 1, à l'action d'un composé de formule $III_A$ :

$CH_3$-CONH-$R_1$ $III_A$

dans laquelle $R_1$ conserve sa signification précédente, pour obtenir un composé de formule $IV_A$ :

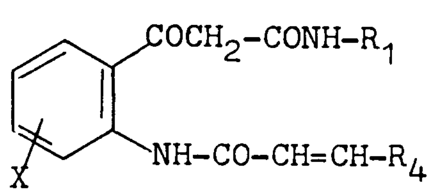

$IV_A$

que l'on cyclise en présence d'un agent alcalin pour obtenir un composé de formule $V_A$ :

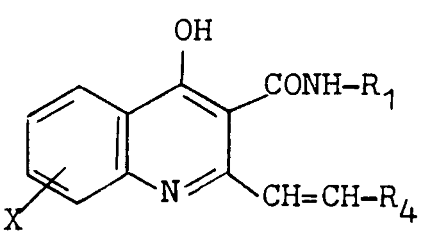

$V_A$

que l'on transforme en un composé de formule (I) dans laquelle X, $R_1$ et $R_4$ ont les significations données à la revendication 1 et $R_2$ et $R_3$ représentent un atome d'hydrogène composé de formule (I)

dont on éthérifie ou estérifie éventuellement une ou les deux fonctions hydroxyle, pour obtenir un composé de formule (I) dans laquelle $R_2$ et/ou $R_3$ représentent soit un radical alcoyle renfermant de 1 à 4 atomes de carbone, un radical phényle, ou un radical

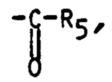

$R_5$ ayant la signification déjà donnée, ou composés de formule (I) dans laquelle $R_2$ et $R_3$ représentent un atome d'hydrogène, que le cas échéant, l'on traite par l'acétone en présence d'un agent acide, pour obtenir l'acétonide correspondant, puis transforme, si désiré, les composés de formule (I) en sels par action d'un acide ou d'une base.

**11.** A titre de médicaments, les composés de formule (I) tels que définis à l'une quelconque des revendications 1 à 7 et leurs sels d'addition avec les acides et les bases pharmaceutiquement acceptables.

**12.** A titre de médicaments, l'un quelconque des composés de formule I, tels que définis à la revendication 8.

**13.** Les compositions pharmaceutiques contenant à titre de principe actif, l'une au moins des médicaments tels que définis à la revendication 11 ou 12.

**14.** A titre de composés industriels nouveaux, les composés de formule II, IV, A, V et VI tels que définis à la revendication 9.

**15.** A titre de composés industriels nouveaux, les composés de formule $II'_A$ correspondant à la formule $II_A$ telle que définie à la revendication 10 dans laquelle $R_4$ a la signification indiquée à la revendication 1 à l'exception du radical phényle, les composés de formules $IV_A$ et $V_A$ tels que définis à la revendication 10.

**Revendication pour l'Etat contactant suivant : AT**

**1.** Les composés de formule (I) :

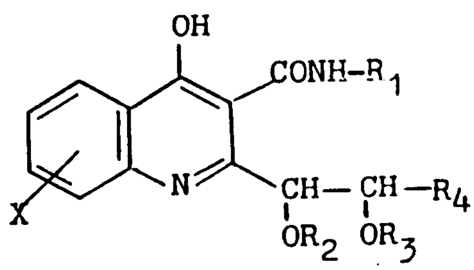

(I)

dans laquelle X en position 5, 6, 7 ou 8 représente un atome d'hydrogène, un atome d'halogène, un radical alcoyle, linéaire ou ramifié, renfermant de 1 à 5 atomes de carbone, un radical alcoxy, linéaire ou ramifié, renfermant de 1 à 4 atomes de carbone, un radical trifluorométhyle, un radical trifluorométhylthio ou un radical trifluorométhoxy, $R_1$ représente un radical choisi parmi les radicaux thiazolyle, 4,5-dihydrothiazolyle, pyridinyle, oxazolyle, isoxazolyle, imidazolyle, pyrimidyle et tétrazolyle, éventuellement substitués par un radical alcoyle renfermant de 1 à 4 atomes de carbone ou $R_1$ représente un radical phényle éventuellement substitué par un ou plusieurs radicaux choisis dans le groupe formé par le radical hydroxy, les radicaux alcoyles renfermant de 1 à 4 atomes de carbone, les radicaux alcoxy renfermant de 1 à 4 atomes de carbone, le radical trifluorométhyle, le radical nitro et les atomes d'halogène, $R_2$ et $R_3$, identiques ou différents, représentent un atome d'hydrogène, un radical alcoyle renfermant de 1 à 4 atomes de carbone, un radical phényle, ou un radical

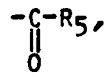

$R_5$ représentant un radical alcoyle renfermant de 1 à 4 atomes de carbone ou un radical phényle, ou $R_2$ et $R_3$ forment ensemble un reste d'acétonide, $R_4$ représente un atome d'hydrogène, un radical alcoyle renfermant de 1 à 4 atomes de carbone ou un radical phényle, ainsi que les sels d'addition des produits de formule (I) avec les acides et les bases.

**2.** Les composés de formule (I), tels que définis à la revendication 1, pour lesquels X est en position 8 ainsi que leurs sels d'addition avec les acides et les bases.

**3.** Les composés de formule (I), tels que définis à la revendication 1 ou 2, pour lesquels X représente un radical trifluorométhyle, ainsi que leurs sels d'addition avec les acides et les bases.

**4.** Les composés de formule (I), tels que définis à la revendication 1, pour lesquels X est en position 7 ainsi que leurs sels d'addition avec les acides et les bases.

**5.** Les composés de formule (I), tels que définis à la revendication 1 ou 4, pour lesquels X représente un atome de chlore, ainsi que leurs sels d'addition avec les acides et les bases.

**6.** Les composés de formule (I), tels que définis à l'une quelconque des revendications 1 à 5, pour lesquels $R_1$ représente le radical thiazolyle, ainsi que leurs sels d'addition avec les acides et les bases.

**7.** Les composés de formule (I) tels que définis à l'une quelconque des revendications 1 à 6, pour lesquels $R_2$ et $R_3$ représentent un atome d'hydrogène et $R_4$ représente un atome d'hydrogène, un radical alcoyle renfermant de 1 à 4 atomes de carbone ou un radical phényle, ainsi que leurs sels d'addition avec les acides et les bases.

**8.** L'un quelconque des composés de formule (I) dont les noms suivent :
- le 2-(1,2-dihydroxy éthyl) 4-hydroxy N-(2-thiazolyl) 8-trifluorométhyl 3-quinoléine carboxamide ainsi que ses sels d'addition avec les acides et les bases,
- le 2-(1,2-dihydroxy propyl) 4-hydroxy N-(2-thiazolyl) 8-trifluorométhyl 3-quinoléine carboxamide ainsi que ses sels d'addition avec les acides et les bases,
- le 2-[1,2-bis (1-oxopropoxy) éthyl] 4-hydroxy N-(2-thiazolyl) 8-trifluorométhyl 3-quinoléine carboxamide ainsi que ses sels d'addition avec les acides et les bases.

**9.** Procédé de préparation des composés de formule (I) telle que définie à la revendication 1 caractérisé en ce que l'on fait réagir un composé de formule II :

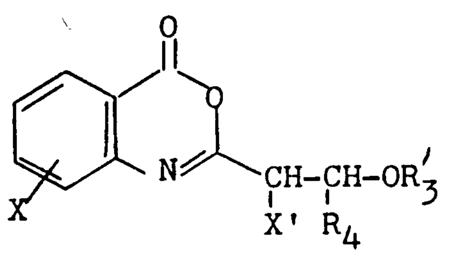

II

dans laquelle X et $R_4$ ont les significations déjà données, $R'_3$ est un radical alcoyle renfermant de 1 à 4 atomes de carbone ou un radical phényle, X' est un atome d'halogène,
- soit avec un composé de formule $III_A$ :

$CH_3$-$CONHR_1$ $III_A$

dans laquelle $R_1$ a la signification précédente, pour obtenir un composé de formule IV :

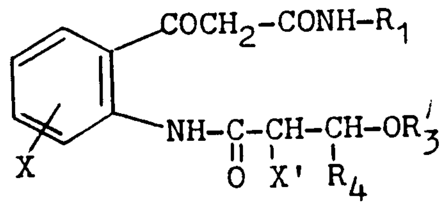

IV

- soit en présence d'une base avec un composé de formule $III_B$ :

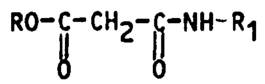

$III_B$

dans laquelle $R_1$ a la signification précédente et R est un radical alcoyle renfermant de 1 à 8 atomes de carbone, pour obtenir un composé de formule (A) :

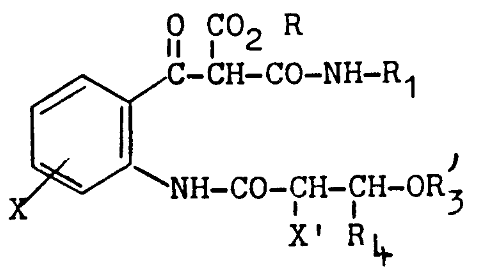

A

que l'on décarbalcoxyle pour obtenir un composé de formule IV précédemment défini, composé de formule IV ainsi obtenu que l'on cyclise en présence d'un agent alcalin pour obtenir un composé de formule V :

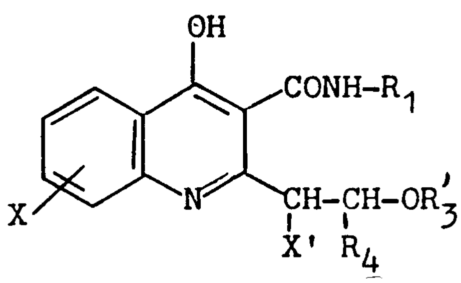

V

que l'on transforme en un composé de formule VI :

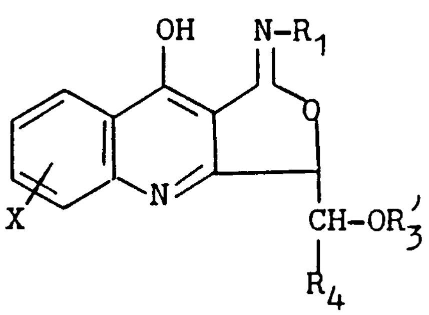

VI

composé de formule VI que l'on traite :

- soit par un agent d'hydrolyse acide pour obtenir un composé de formule (I) dans laquelle X, $R_1$ et $R_4$ ont les significations précédentes, $R_2$ est un atome d'hydrogène et $R_3$ un radical alcoyle

renfermant de 1 à 4 atomes de carbone ou un radical phényle, que l'on transforme, si désiré, en composé de formule (I) dans laquelle X, $R_1$, $R_2$ et $R_4$ ont les significations données ci-dessus et $R_3$ représente un atome d'hydrogène, puis, le cas échéant, éthérifie ou estérifie une ou les deux fonctions hydroxyle, pour obtenir un composé de formule (I) dans laquelle $R_2$ et/ou $R_3$, identiques ou différents, représentent un radical alcoyle renfermant de 1 à 4 atomes de carbone, un radical phényle ou un groupement

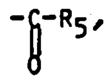

tel que défini précédemment, ou, le cas échéant, traite un composé de formule (I) dans laquelle $R_2$ et $R_3$ représentent un atome d'hydrogène par l'acétone en présence d'un agent acide, pour obtenir l'acétonide correspondant,

- soit par un acide de formule $R_5$-COOH, $R_5$ ayant la signification précédente, pour obtenir un produit de formule (I) dans laquelle X, $R_1$ et $R_4$ ont les significations précédentes, $R_2$ représente un radical

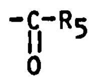

et $R_3$ un radical alcoyle renfermant de 1 à 4 atomes de carbone ou un radical phényle, composés de formule (I) ainsi obtenus, que l'on transforme, si désiré, en sels par action d'un acide ou d'une base.

**Claims**

**Claims for the following Contracting States : BE, CH, DE, FR, GB, IT, LI, LU, NL, SE**

**1.** The compounds of formula (I):

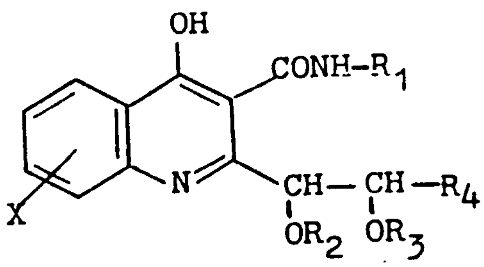

(I)

in which X in position 5, 6, 7 or 8 represents a hydrogen atom, a halogen atom, a linear or branched alkyl radical containing 1 to 5 carbon atoms, a linear or branched alkoxy radical containing 1 to 4 carbon atoms, a trifluoromethyl radical, a trifluoromethylthio radical or a trifluoromethoxy radical, $R_1$ represents a radical chosen from the following radicals: thiazolyl, 4,5-dihydrothiazolyl, pyridinyl, oxazolyl, isoxazolyl, imidazolyl, pyrimidyl and tetrazolyl, optionally substituted by an alkyl radical containing 1 to 4 carbon atoms or $R_1$ represents a phenyl radical optionally substituted by one or more radicals chosen from the group formed by the following radicals: hydroxy, alkyl containing 1 to 4 carbon atoms, alkoxy containing 1 to 4 carbon atoms, trifluoromethyl, nitro and halogen atoms, $R_2$ and $R_3$, identical or different, represent a hydrogen atom, an alkyl radical containing 1 to 4 carbon atoms, a phenyl radical or a

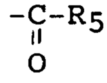

radical, $R_5$ representing an alkyl radical containing 1 to 4 carbon atoms or a phenyl radical, or $R_2$ and

$R_3$ together form an acetonide remainder, $R_4$ represents a hydrogen atom, an alkyl radical containing 1 to 4 carbon atoms or a phenyl radical, as well as the addition salts of the products of formula (I) with acids and bases.

**2.** The compounds of formula (I), as defined in claim 1, for which X is in position 8 as well as their addition salts with acids and bases.

**3.** The compounds of formula (I), as defined in claim 1 or 2, for which X represents a trifluoromethyl radical, as well as their addition salts with acids and bases.

**4.** The compounds of formula (I), as defined in claim 1, for which X is in position 7, as well as their addition salts with acids and bases.

**5.** The compounds of formula (I), as defined in claim 1 or 4, for which X represents a chlorine atom, as well as their addition salts with acids and bases.

**6.** The compounds of formula (I) as defined in any one of claim 1 to 5, for which $R_1$ represents a thiazolyl radical as well as their addition salts with acids and bases.

**7.** The compounds of formula (I), as defined in any one of claims 1 to 6, for which $R_2$ and $R_3$ represent a hydrogen atom and $R_4$ represents a hydrogen atom, an alkyl radical containing 1 to 4 carbon atoms or a phenyl radical, as well as their addition salts with acids and bases.

**8.** Any one of the compounds of formula (I) the names of which follow:
- 2-(1,2-dihydroxy ethyl) 4-hydroxy N-(2-thiazolyl) 8-trifluoromethyl 3-quinoline carboxamide as well as its addition salts with acids and bases,
- 2-(1,2-dihydroxy propyl) 4-hydroxy N-(2-thiazolyl) 8-trifluoromethyl 3-quinoline carboxamide as well as its addition salts with acids and bases,
- 2-[1,2-bis (1-oxopropoxy) ethyl] 4-hydroxy N-(2-thiazolyl) 8-trifluoromethyl 3-quinoline carboxamide as well as its addition salts with acids and bases.

**9.** Preparation process for the compounds of formula (I) as defined in claim 1, characterized in that a compound of formula (II):

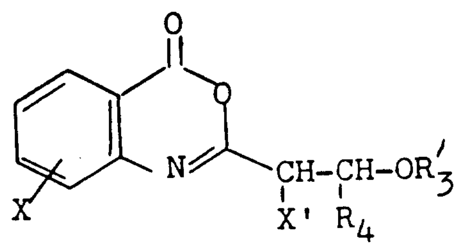

(II)

in which X and $R_4$ have the meanings already given, $R'_3$ is an alkyl radical containing 1 to 4 carbon atoms or a phenyl radical, X' is a halogen atom, is reacted,
- either with a compound of formula ($III_A$):

$CH_3$-$CONHR_1$ ($III_A$)

in which $R_1$ has the previous meaning, in order to obtain a compound of formula (IV):

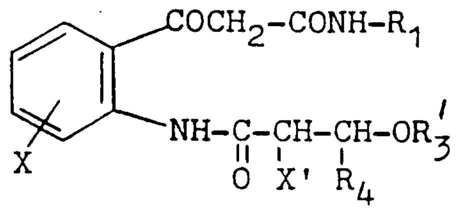

(IV)

- or with a compound of formula ($III_B$) in the presence of a base:

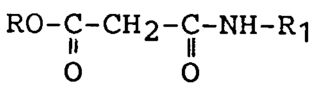

($III_B$)

in which $R_1$ has the previous meaning and R is an alkyl radical containing 1 to 8 carbon atoms, in order to obtain a compound of formula (A):

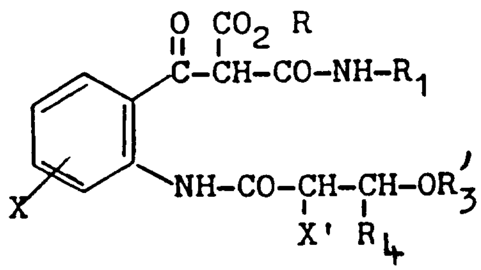

(A)

which is decarbalkoxylated in order to obtain a compound of formula (IV) defined previously, which compound of formula IV thus obtained is cyclized in the presence of an alkaline agent in order to obtain a compound of formula (V):

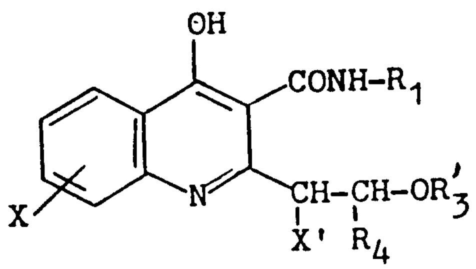

(V)

which is converted into a compound of formula (VI):

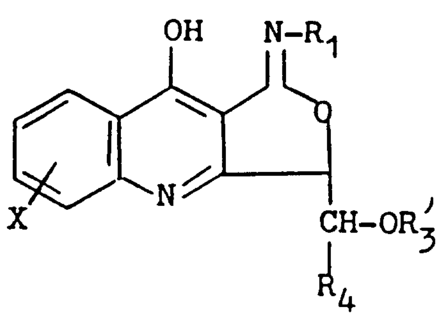

(VI)

which compound of formula (VI) is treated:

- either with a hydrolysis agent in order to obtain a compound of formula (I) in which X, $R_1$ and $R_4$ have the previous meanings, $R_2$ is a hydrogen atom and $R_3$ is an alkyl radical containing 1 to 4 carbon atoms or a phenyl radical, which is converted, if desired, into a compound of formula (I) in which X, $R_1$, $R_2$ and $R_4$ have the meanings given above and $R_3$ represents a hydrogen atom, then, if appropriate, one or both of the hydroxyl functions are etherified or esterified in order to obtain a compound of formula (I) in which $R_2$ and/or $R_3$, identical or different, represent an alkyl radical containing 1 to 4 carbon atoms, a phenyl radical or a

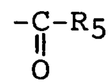

group as defined previously, or, if appropriate, a compound of formula (I) in which $R_2$ and $R_3$ represent a hydrogen atom is treated with acetone in the presence of an acid agent, in order to obtain the corresponding acetonide,
- or with an acid of formula $R_5$-COOH, $R_5$ having the previous meaning, in order to obtain a product of formula (I) in which X, $R_1$ and $R_4$ have the previous meanings, $R_2$ represents a

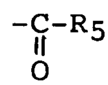

radical and $R_3$ an alkyl radical containing 1 to 4 carbon atoms or a phenyl radical, which compounds of formula (I) thus obtained are converted, if desired, into salts by the action of an acid or a base.

**10.** Preparation process for the compounds of formula (I) as defined in claim 1, characterized in that a compound of formula ($II_A$):

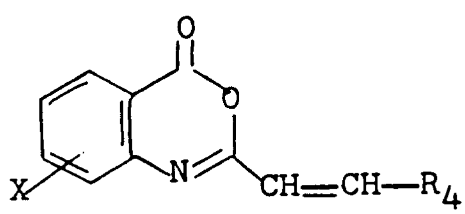

($II_A$)

X and $R_4$ having the meanings indicated in claim 1, is subjected to the action of a compound of formula ($III_A$):

$CH_3$-CONH-$R_1$ ($III_A$)

in which $R_1$ keeps its previous meaning, in order to obtain a compound of formula ($IV_A$):

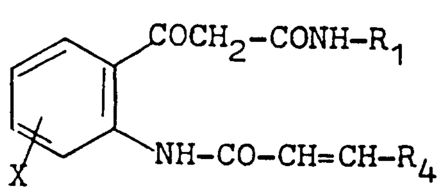

($IV_A$)

which is cyclized in the presence of an alkaline agent in order to obtain a compound of formula ($V_A$):

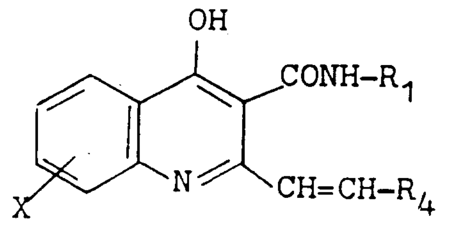

$(V_A)$

which is converted into a compound of formula (I) in which X, $R_1$ and $R_4$ have the meanings given in claim 1 and $R_2$ and $R_3$ represent a hydrogen atom, one or both hydroxyl functions of which compound of formula (I) are optionally etherified or esterified, in order to obtain a compound of formula (I) in which $R_2$ and/or $R_3$ represent either an alkyl radical containing 1 to 4 carbon atoms, a phenyl radical or a

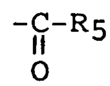

radical, $R_5$ having the meaning already given, or compounds of formula (I) in which $R_2$ and $R_3$ represent a hydrogen atom, which, if appropriate, are treated with acetone in the presence of an acid agent, in order to obtain the corresponding acetonide, then if desired, the compounds of formula (I) are converted into salts by the action of an acid or a base.

**11.** As medicaments, the compounds of formula (I) as defined in any one of claims 1 to 7 and their addition salts with pharmaceutically acceptable acids and bases.

**12.** As medicaments, any one of the compounds of formula (I) as defined in claim 8.

**13.** The pharmaceutical compositions containing as active ingredient at least one of the medicaments as defined in claim 11 or 12.

**14.** As new industrial compounds, the compounds of formulae (II), (IV), (A), (V) and (VI) as defined in claim 9.

**15.** As new industrial compounds, the compounds of formula $(II'_A)$ corresponding to formula $(II_A)$ as defined in claim 10, in which $R_4$ has the meaning indicated in claim 1 with the exception of the phenyl radical, the compounds of formulae $(IV_A)$ and $(V_A)$ as defined in claim 10.

**Claim for the following Contracting State : AT**

**1.** The compounds of formula (I):

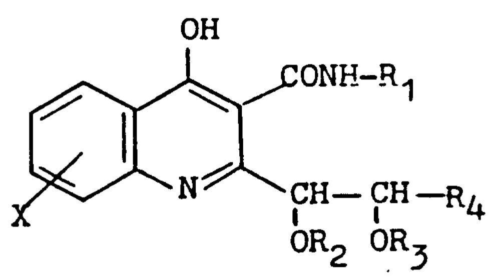

(I)

in which X in position 5, 6, 7 or 8 represents a hydrogen atom, a halogen atom, a linear or branched alkyl radical containing 1 to 5 carbon atoms, a linear or branched alkoxy radical containing 1 to 4 carbon atoms, a trifluoromethyl radical, a trifluoromethylthio radical or a trifluoromethoxy radical, $R_1$ represents a radical chosen from the following radicals: thiazolyl, 4,5-dihydrothiazolyl, pyridinyl, oxazolyl, isoxazolyl, imidazolyl, pyrimidyl and tetrazolyl, optionally substituted by an alkyl radical containing 1 to 4 carbon atoms or $R_1$ represents a phenyl radical optionally substituted by one or more radicals chosen from the group formed by the following radicals: hydroxy, alkyl containing 1 to 4 carbon atoms, alkoxy containing 1 to 4 carbon atoms, trifluoromethyl, nitro and halogen atoms, $R_2$ and $R_3$, identical or different, represent a hydrogen atom, an alkyl radical containing 1 to 4 carbon atoms, a phenyl radical or a

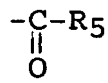

radical, $R_5$ representing an alkyl radical containing 1 to 4 carbon atoms or a phenyl radical, or $R_2$ and $R_3$ together form an acetonide remainder, $R_4$ represents a hydrogen atom, an alkyl radical containing 1 to 4 carbon atoms or a phenyl radical, as well as the addition salts of the products of formula (I) with acids and bases.

**2.** The compounds of formula (I), as defined in claim 1, for which X is in position 8 as well as their addition salts with acids and bases.

**3.** The compounds of formula (I), as defined in claim 1 or 2, for which X represents a trifluoromethyl radical, as well as their addition salts with acids and bases.

**4.** The compounds of formula (I), as defined in claim 1, for which X is in position 7, as well as their addition salts with acids and bases.

**5.** The compounds of formula (I), as defined in claim 1 or 4, for which X represents a chlorine atom, as well as their addition salts with acids and bases.

**6.** The compounds of formula (I), as defined in any one of claims 1 to 5, for which $R_1$ represents a thiazolyl radical, as well as their addition salts with acids and bases.

**7.** The compounds of formula (I) as defined in any one of claim 1 to 6 for which $R_2$ and $R_3$ represent a hydrogen atom and $R_4$ represents a hydrogen atom, an alkyl radical containing 1 to 4 carbon atoms or a phenyl radical, as well as their addition salts with acids and bases.

**8.** Any one of the compounds of formula (I) the names of which follow:

- 2-(1,2-dihydroxy ethyl) 4-hydroxy N-(2-thiazolyl) 8-trifluoromethyl 3-quinoline carboxamide as well as its addition salts with acids and bases,
- 2-(1,2-dihydroxy propyl) 4-hydroxy N-(2-thiazolyl) 8-trifluoromethyl 3-quinoline carboxamide as well as its addition salts with acids and bases,
- 2-[1,2-bis (1-oxopropoxy) ethyl] 4-hydroxy N-(2-thiazolyl) 8-trifluoromethyl 3-quinoline carboxamide as well as its addition salts with acids and bases.

**9.** Preparation process for the compounds of formula (I) as defined in claim 1, characterized in that a compound of formula (II):

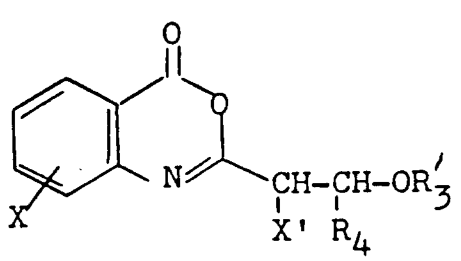

(II)

in which X and $R_4$ have the meanings already given, $R'_3$ is an alkyl radical containing 1 to 4 carbon

atoms or a phenyl radical, X' is a halogen atom, is reacted,

- either with a compound of formula ($III_A$):

$CH_3$-CONHR$_1$ ($III_A$)

in which $R_1$ has the previous meaning, in order to obtain a compound of formula (IV):

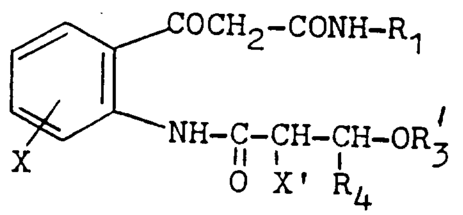

(IV)

- or with a compound of formula ($III_B$) in the presence of a base:

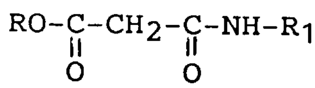

($III_B$)

in which $R_1$ has the previous meaning and R is an alkyl radical containing 1 to 8 carbon atoms, in order to obtain a compound of formula (A):

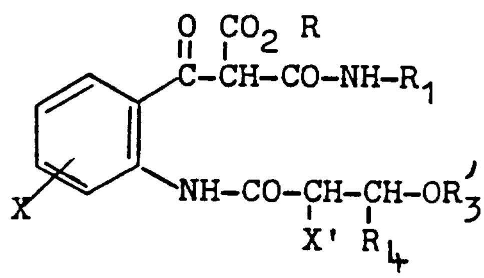

(A)

which is decarbalkoxylated in order to obtain a compound of formula (IV) defined previously, which compound of formula IV thus obtained is cyclized in the presence of an alkaline agent in order to obtain a compound of formula (V):

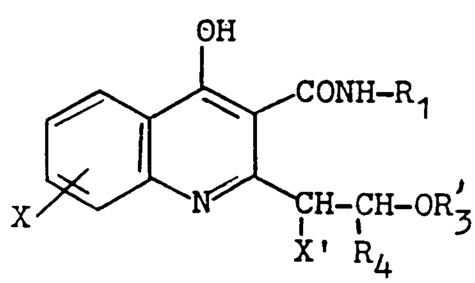

(V)

which is converted into a compound of formula (VI):

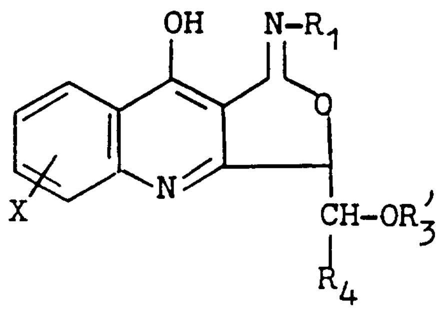

(VI)

which compound of formula (VI) is treated:

- either with a hydrolysis agent in order to obtain a compound of formula (I) in which X, $R_1$ and $R_4$ have the previous meanings, $R_2$ is a hydrogen atom and $R_3$ is an alkyl radical containing 1 to 4 carbon atoms or a phenyl radical, which is converted, if desired, into a compound of formula (I) in which X, $R_1$, $R_2$ and $R_4$ have the meanings given above and $R_3$ represents a hydrogen atom, then, if appropriate, one or both of the hydroxyl functions are etherified or esterified in order to obtain a compound of formula (I) in which $R_2$ and/or $R_3$, identical or different, represent an alkyl radical containing 1 to 4 carbon atoms, a phenyl radical or a

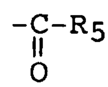

group as defined previously, or, if appropriate, a compound of formula (I) in which $R_2$ and $R_3$ represent a hydrogen atom is treated with acetone in the presence of an acid agent, in order to obtain the corresponding acetonide,
- or with an acid of formula $R_5$-COOH, $R_5$ having the previous meaning, in order to obtain a compound of formula (I) in which X, $R_1$ and $R_4$ have the previous meanings, $R_2$ represents a

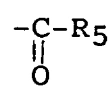

radical and $R_3$ an alkyl radical containing 1 to 4 carbon atoms or a phenyl radical, which compounds of formula (I) thus obtained are converted, if desired, into salts by the action of an acid or a base.

**Patentansprüche**

**Patentansprüche für folgende Vertragstaaten : BE, CH, DE, FR, GB, IT, LI, LU, NL, SE**

**1.** Verbindungen der Formel (I)

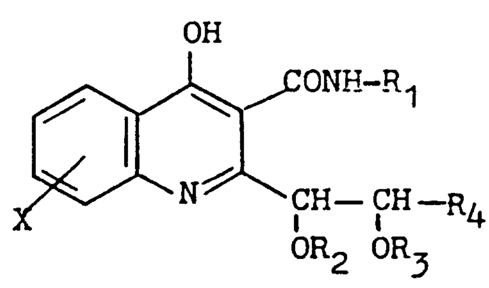

(I)

worin X in 5-, 6-, 7- oder 8-Stellung ein Wasserstoffatom, Halogenatom, einen linearen oder verzweigten Alkylrest mit 1 bis 5 Kohlenstoffatomen und einen linearen oder verzweigten Alkoxyrest mit 1 bis 4

Kohlenstoffatomen, einen Trifluormethylrest, einen Trifluormethylthiorest oder einen Trifluormethoxyrest bedeutet, $R_1$ für einen Rest steht, ausgewählt unter den Thiazolyl-, 4-5-Dihydrothiazolyl-, Pyrridinyl-, Oxazolyl-, Isoxazolyl-, Imidazolyl-, Pyrimidyl- und Tetrazolylresten, die gegebenenfalls substituiert sind durch einen Alkylrest mit 1 bis 4 Kohlenstoffatomen, oder $R_1$ für einen Phenylrest steht, der gegebenenfalls durch einen oder mehrere Reste substituiert ist, ausgewählt unter der Hydroxygruppe, den Alkylresten mit 1 bis 4 Kohlenstoffatomen, den Alkoxyresten mit 1 bis 4 Kohlenstoffatomen, der Trifluormethylgruppe, der Nitrogruppe und den Halogenatomen, $R_2$ und $R_3$, die gleich oder voneinander verschieden sind, ein Wasserstoffatom, einen Alkylrest mit 1 bis 4 Kohlenstoffatomen, einen Phenylrest oder einen Rest

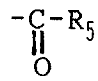

bedeuten, wobei $R_5$ für einen Alkylrest mit 1 bis 4 Kohlenstoffatomen oder einen Phenylrest steht, oder $R_2$ und $R_3$ gemeinsam einen Acetonidrest bilden, $R_4$ ein Wasserstoffatom, eine Alkylgruppe mit 1 bis 4 Kohlenstoffatomen oder einen Phenylrest bedeutet, sowie die Additionssalze der Produkte der Formel (I) mit Säuren und Basen.

**2.** Verbindungen der Formel (I), wie in Anspruch 1 definiert, worin sich X in 8-Stellung befindet, sowie deren Additionssalze mit Säuren und Basen.

**3.** Verbindungen der Formel (I), wie in Anspruch 1 oder 2 definiert, worin X einen Trifluormethylrest bedeutet, sowie deren Additionssalze mit Säuren und Basen.

**4.** Verbindungen der Formel (I), wie in Anspruch 1 definiert, worin X sich in 7-Stellung befindet, sowie deren Additionssalze mit Säuren und Basen.

**5.** Verbindungen der Formel (I), wie in Anspruch 1 oder 4 definiert, worin X ein Chloratom bedeutet, sowie deren Additionssalze mit Säuren und Basen.

**6.** Verbindungen der Formel (I), wie in einem der Ansprüche 1 bis 5 definiert, worin $R_1$ für den Thiazolylrest steht, sowie deren Additionssalze mit Säuren und Basen.

**7.** Verbindungen der Formel (I), wie in einem der Ansprüche 1 bis 6 definiert, worin $R_2$ und $R_3$ ein Wasserstoffatom bedeuten, und $R_4$ ein Wasserstoffatom, einen Alkylrest mit 1 bis 4 Kohlenstoffatomen oder einen Phenylrest bedeutet, sowie deren Additionssalze mit Säuren und Basen.

**8.** Eine der Verbindungen der Formel (I) mit den folgenden Bezeichnungen:
- 2-(1,2-Dihydroxyethyl)-4-hydroxy-N-(2-thiazolyl)-8-trifluormethyl-3-chinolin-carboxamid, sowie dessen Additionssalze mit Säuren und Basen,
- 2-(1,2-Dihydroxypropyl)-4-hydroxy-N-(2-thiazolyl)-8-trifluormethyl-3-chinolin-carboxamid, sowie dessen Additionsalze mit Säuren und Basen,
- 2-[1,2-bis-(1-Oxopropoxy)-ethyl]-4-hydroxy-N-(2-thiazolyl)-8-trifluormethyl-3-chinolin-carboxamid, sowie dessen Additionssalze mit Säuren und Basen.

**9.** Verfahren zur Herstellung der Verbindungen der Formel (I), wie in Anspruch 1 definiert, dadurch gekennzeichnet, daß man eine Verbindung der Formel (II)

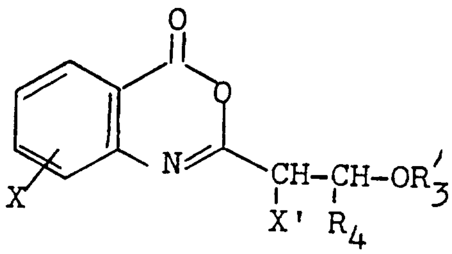

II

worin X und $R_4$ die angegebenen Bedeutungen besitzen, $R'_3$ einen Alkylrest mit 1 bis 4 Kohlenstoffatomen oder einen Phenylrest bedeutet, X' für ein Halogenatom steht,

- entweder mit einer Verbindung der Formel ($III_A$)

$CH_3$-$CONHR_1$ $III_A$

worin $R_1$ die vorstehende Bedeutung besitzt, umsetzt, um eine Verbindung der Formel (IV)

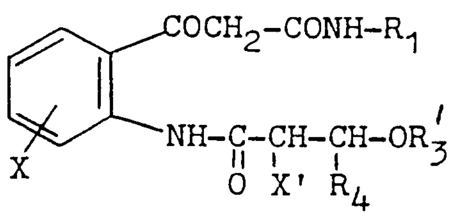

IV

zu erhalten,

- oder in Anwesenheit einer Base mit einer Verbindung der Formel ($III_B$)

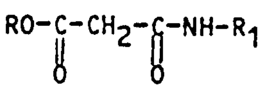

$III_B$

worin $R_1$ die vorstehende Bedeutung besitzt, und R für einen Alkylrest mit 1 bis 8 Kohlenstoffatomen steht, umsetzt, um eine Verbindung der Formel (A)

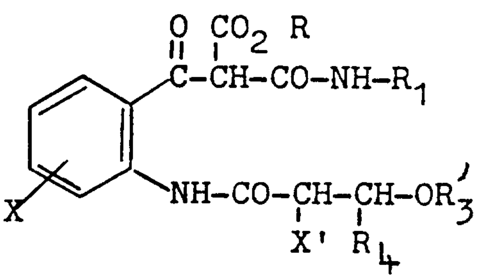

A

zu erhalten, die man decarbalkoxiliert, um eine Verbindung der vorstehend definierten Formel (IV) zu erhalten, die so erhaltene Verbindung der Formel (IV) in Anwesenheit eines alkalischen Mittels cyclisiert, um zu einer Verbindung der Formel (V)

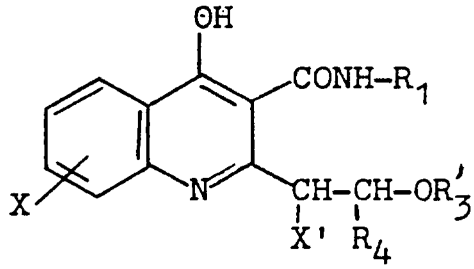

V

zu gelangen, welche man in eine Verbindung der Formel (VI)

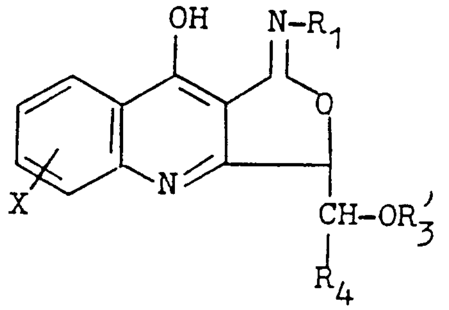

VI

überführt, man die Verbindung der Formel (VI) behandelt:

- entweder mit einem sauren Hydrolysemittel, um eine Verbindung der Formel (I) zu erhalten, worin X, $R_1$ und $R_4$ die vorstehenden Bedeutungen besitzen, $R_2$ ein Wasserstoffatom ist, und $R_3$ einen Alkylrest mit 1 bis 4 Kohlenstoffatomen oder einen Phenylrest darstellt, welche man gewünschtenfalls in eine Verbindung der Formel (I) überführt, worin X, $R_1$, $R_2$ und $R_4$ die vorstehend angegebenen Bedeutungen besitzen, und $R_3$ ein Wasserstoffatom darstellt, wonach man gegebenenfalls eine oder die beiden Hydroxylfunktionen verethert oder verestert, um zu einer Verbindung der Formel (I) zu gelangen, worin $R_2$ und/oder $R_3$, die gleich oder voneinander verschieden sind, einen Alkylrest mit 1 bis 4 Kohlenstoffatomen, einen Phenylrest oder eine Gruppe

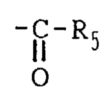

wie vorstehend definiert, bedeuten, oder gegebenenfalls eine Verbindung der Formel (I), worin $R_2$ und $R_3$ ein Wasserstoffatom bedeuten, mit Aceton in Anwesenheit eines sauren Mittels behandelt, um das entsprechende Acetonid zu erhalten,
- oder mit einer Säure der Formel $R_5$-COOH, worin $R_5$ die vorstehende Bedeutung besitzt, um ein Produkt der Formel (I) zu erhalten, worin X, $R_1$ und $R_4$ die vorstehenden Bedeutungen besitzen, $R_2$ ein Rest

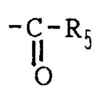

bedeutet, und $R_3$ einen Alkylrest mit 1 bis 4 Kohlenstoffatomen oder einen Phenylrest darstellt, und die so erhaltenen Verbindungen der Formel (I) man gewünschtenfalls durch Einwirken einer Säure oder einer Base in Salze überführt.

**10.** Verfahren zur Herstellung der Verbindungen der Formel (I), wie in Anspruch 1 definiert, dadurch gekennzeichnet, daß man eine Verbindung der Formel ($II_A$)

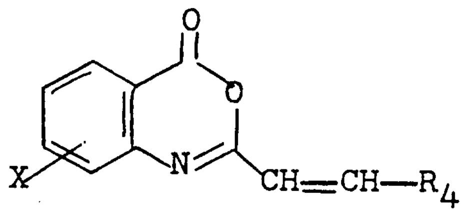

$II_A$

worin X und $R_4$ die in Anspruch 1 angegebenen Bedeutungen besitzen, der Einwirkung einer Verbindung der Formel ($III_A$)

$CH_3$-CONH-$R_1$ $III_A$

unterzieht, worin $R_1$ die vorstehende Bedeutung besitzt, um zu einer Verbindung der Formel ($IV_A$)

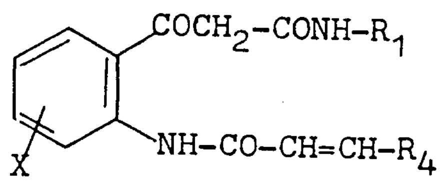

$IV_A$

zu gelangen, die man in Gegenwart eines alkalischen Mittels cyclisiert, um zu einer Verbindung der Formel ($V_A$)

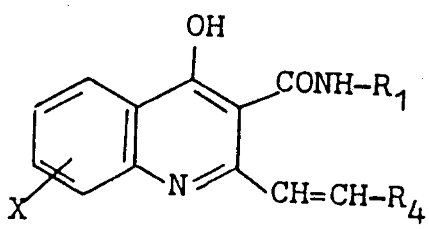

$V_A$

zu gelangen, die man in eine Verbindung der Formel (I) überführt, worin X, $R_1$ und $R_4$ die in Anspruch 1 angegebenen Bedeutungen besitzen, und $R_2$ und $R_3$ ein Wasserstoffatom bedeuten, man eine oder beide Hydroxylfunktionen der Verbindungen der Formel (I) gegebenenfalls verethert oder verestert, um eine Verbindung der Formel (I) zu erhalten, worin $R_2$ und/oder $R_3$ entweder einen Alkylrest mit 1 bis 4 Kohlenstoffatomen, einen Phenylrest oder einen Rest

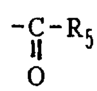

bedeuten, worin $R_5$ die vorstehend angegebene Bedeutung besitzt, oder man die Verbindungen der Formel (I), worin $R_2$ und $R_3$ ein Wasserstoffatom bedeuten, gegebenenfalls mit Aceton in Anwesenheit eines sauren Mittels behandelt, um zu dem entsprechenden Acetonid zu gelangen, wonach man gewünschtenfalls die Verbindungen der Formel (I) durch Einwirken einer Säure oder einer Base in Salze überführt.

**11.** Als Arzneimittel die Verbindungen der Formel (I), wie in einem der Ansprüche 1 bis 7 definiert, und ihre Additionssalze mit pharmazeutisch verträglichen Säuren und Basen.

**12.** Als Arzneimittel eine der Verbindungen der Formel (I), wie in Anspruch 8 definiert.

**13.** Pharmazeutische Zusammensetzungen, enthaltend als Wirkstoff zumindest eines der Arzneimittel, wie in Anspruch 11 oder 12 definiert.

**14.** Als neue industrielle Verbindungen die Verbindungen der Formel (II), (IV), (A), (V) und (VI), wie in Anspruch 9 definiert.

**15.** Als neue industrielle Verbindungen die Verbindungen der Formel ($II'_A$), entsprechend der Formel ($II_A$), wie in Anspruch 10 definiert, worin $R_4$ die in Anspruch 1 angegebene Bedeutung mit Ausnahme des Phenylrestes besitzt, die Verbindungen der Formel ($IV_A$) und ($V_A$), wie in Anspruch 10 definiert.

**Patentanspruch für folgende Vertragsstaat : AT**

**1.** Verbindungen der Formel (I)

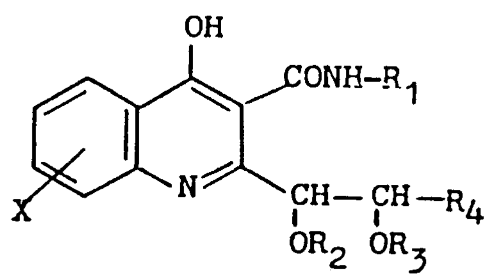

(I)

worin X in 5-, 6-, 7- oder 8-Stellung ein Wasserstoffatom, ein Halogenatom, einen linearen oder verzweigten Alkylrest mit 1 bis 5 Kohlenstoffatomen, einen linearen oder verzweigten Alkoxyrest mit 1 bis 4 Kohlenstoffatomen, eine Trifluormethylgruppe, eine Trifluormethylthiogruppe oder einen Trifluormethoxyrest bedeutet, $R_1$ für einen Rest steht, ausgewählt unter den Thiazolyl-, 4,5-Dihydrothiazolyl-, Pyrridinyl-, Oxazolyl-, Isoxazolyl-, Imidazolyl-, Pyrimidyl- und Tetrazolylresten, gegebenenfalls substituiert durch einen Alkylrest mit 1 bis 4 Kohlenstoffatomen, oder $R_1$ einen Phenylrest bedeutet, der gegebenenfalls substituiert ist durch einen oder mehrere Reste, ausgewählt unter der Hydroxygruppe, den Alkylresten mit 1 bis 4 Kohlenstoffatomen, den Alkoxyresten mit 1 bis 4 Kohlenstoffatomen, der Trifluormethylgruppe, der Nitrogruppe und den Halogenatomen, $R_2$ und $R_3$, die identisch oder voneinander verschieden sind, ein Wasserstoffatom, eine Alkylgruppe mit 1 bis 4 Kohlenstoffatomen, einen Phenylrest oder einen Rest

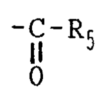

bedeuten, worin $R_5$ einen Alkylrest mit 1 bis 4 Kohlenstoffatomen oder einen Phenylrest darstellt, oder $R_2$ und $R_3$ gemeinsam einen Acetonidrest bilden, $R_4$ für ein Wasserstoffatom, eine Alkylgruppe mit 1 bis 4 Kohlenstoffatomen oder einen Phenylrest steht, sowie die Additionssalze der Produkte der Formel (I) mit Säuren und Basen.

**2.** Die Verbindungen der Formel (I), wie in Anspruch 1 definiert, worin X in 8-Stellung vorliegt, sowie deren Additionssalze mit Säuren und Basen.

**3.** Die Verbindungen der Formel (I), wie in Anspruch 1 oder 2 definiert, worin X einen Trifluormethylrest bedeutet, sowie deren Additionssalze mit Säuren und Basen.

**4.** Die Verbindungen der Formel (I), wie in Anspruch 1 definiert, worin X sich in 7-Stellung befindet, sowie deren Additionssalze mit Säuren und Basen.

**5.** Die Verbindungen der Formel (I), wie in Anspruch 1 oder 4 definiert, worin X ein Chloratom bedeutet, sowie deren Additionssalze mit Säuren und Basen.

**6.** Die Verbindungen der Formel (I), wie in einem der Ansprüche 1 bis 5 definiert, worin $R_1$ den Thiazolylrest bedeutet, sowie deren Additionssalze mit Säuren und Basen.

**7.** Die Verbindungen der Formel (I), wie in einem der Ansprüche 1 bis 6 definiert, worin $R_2$ und $R_3$ für ein Wasserstoffatom stehen, und $R_4$ ein Wasserstoffatom, einen Alkylrest mit 1 bis 4 Kohlenstoffatomen oder einen Phenylrest bedeutet, sowie deren Additionssalze mit Säuren und Basen.

**8.** Eine der Verbindungen der Formel (I) mit den folgenden Bezeichnungen:
- 2-(1-2-Dihydroxyethyl)-4-hydroxy-N-(2-thiazolyl)-8-trifluormethyl-3-chinolin-carboxamid, sowie dessen Additionssalze mit Säuren und Basen,
- 2-(1,2-Dihydroxypropyl)-4-hydroxy-N-(2-thiazolyl)-8-trifluormethyl-3-chinolin-carboxamid, sowie dessen Additionssalze mit Säuren und Basen,
- 2-[1,2-bis-(1-Oxonpropxy)-ethyl]-4-hydroxy-N-(2-thiazolyl)-8-trifluormethyl-3-chinolin-carbonamid, sowie dessen Additionssalze mit Säuren und Basen.

**9.** Verfahren zur Herstellung der Verbindungen der Formel (I), wie in Anspruch 1 definiert, dadurch gekennzeichnet, daß man eine Verbindung der Formel (II)

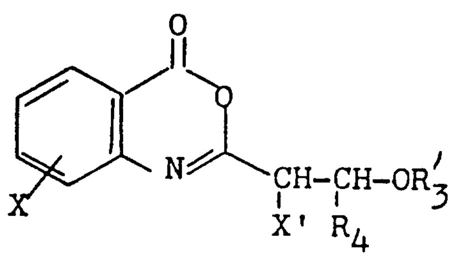

II

worin X und $R_4$ die angegebenen Bedeutungen besitzen, $R'_3$ einen Alkylrest mit 1 bis 4 Kohlenstoffatomen oder einen Phenylrest wiedergibt, X' für ein Halogenatom steht,
- entweder mit einer Verbindung der Formel ($III_A$)

$CH_3$-$CONHR_1$ IIIA

umsetzt, worin $R_1$ die vorstehende Bedeutung besitzt, um eine Verbindung der Formel (IV)

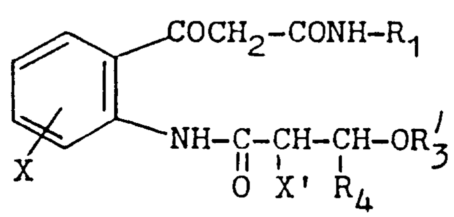

IV

zu erhalten,

- oder in Gegenwart einer Base mit einer Verbindung der Formel

($III_B$)

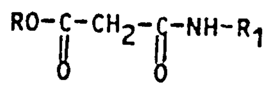

$III_B$

worin $R_1$ die vorstehende Bedeutung besitzt, und R für einen Alkylrest mit 1 bis 8 Kohlenstoffatomen steht, umsetzt, um eine Verbindung der Formel (A)

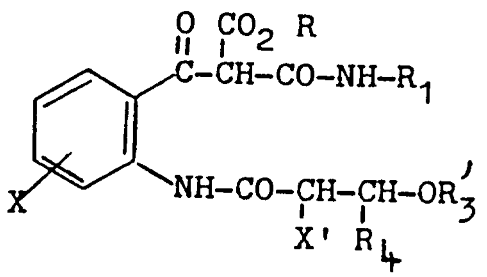

A

zu erhalten, die man decarbalkoxiliert, um zu einer Verbindung der vorstehend definierten Formel (IV) zu gelangen, die so erhaltene Verbindung der Formel (IV) man in Anwesenheit eines alkalischen Mittels cyclisiert, um zu einer Verbindung der Formel (V)

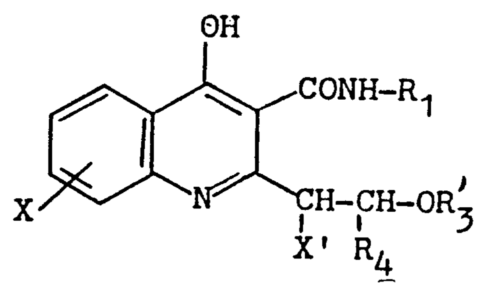

V

zu gelangen, welche man in eine Verbindung der Formel (VI)

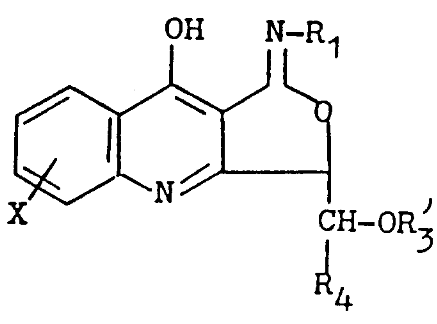

VI

überführt, man die Verbindung der Formel (VI) behandelt:

- entweder mit einem sauren Hydrolysemittel, um eine Verbindung der Formel (I) zu erhalten, worin X, $R_1$ und $R_4$ die vorstehenden Bedeutungen besitzen, $R_2$ ein Wasserstoffatom bedeutet, und $R_3$ für einen Alkylrest mit 1 bis 4 Kohlenstoffatomen oder einen Phenylrest steht, welche man gewünschtenfalls in eine Verbindung der Formel (I) überführt, worin X, $R_1$, $R_2$ und $R_4$ die vorstehend angegebenen Bedeutungen besitzen, und $R_3$ ein Wasserstoffatom bedeutet, wonach man gegebenenfalls eine oder beide Hydroxylfunktionen verethert oder verestert, um eine

Verbindung der Formel (I) zu erhalten, worin $R_2$ und/oder $R_3$, die gleich oder voneinander verschieden sind, einen Alkylrest mit 1 bis 4 Kohlenstoffatomen, einen Phenylrest oder eine Gruppe

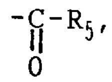

wie vorstehend definiert, bedeuten, oder gegebenenfalls eine Verbindung der Formel (I), worin $R_2$ und $R_3$ ein Wasserstoffatom bedeuten, mit Aceton in Gegenwart eines sauren Mittels behandelt, um zu dem entsprechendem Acetonid zu gelangen,

- oder mit einer Säure der Formel $R_5$-COOH, worin $R_5$ die vorstehende Bedeutung besitzt, behandelt, um zu einem Produkt der Formel (I) zu gelangen, worin X, $R_1$ und $R_4$ die vorstehenden Bedeutungen besitzen, $R_2$ einen Rest

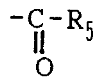

wiedergibt, und $R_3$ einen Alkylrest mit 1 bis 4 Kohlenstoffatomen oder einen Phenylrest bedeutet, und die so erhaltenen Verbindungen der Formel (I) gewünschtenfalls durch Umsetzung mit einer Säure oder mit einer Base in Salze überführt.